# EUROPEAN PATENT SPECIFICATION

(11) **EP 2 676 953 B1**
(45) Date of publication and mention of the grant of the patent: **22.03.2017**
(21) Application number: 13177190.9
(22) Date of filing: 16.12.2009
(51) Int. Cl.: C07D 205/04, A61K 31/397, A61P 35/00, A61P 37/00

(54) **HEMIFUMARATE SALT OF 1-[4-[1-(4-CYCLOHEXYL-3 -TRIFLUOROMETHYL-BENZYLOXYIMINO)-ETHYL]-2-ETHYL-BENZYL]-AZETIDINE-3-CARBOXYLIC ACID FOR USE IN THE TREATMENT OF LYMPHOCYTE MEDIATED DISEASES**
HEMIFUMARATSALZ VON 1-[4-[1-(4-CYCLOHEXYL-3-TRIFLUORMETHYLBENZYLOXYIMINO)-ETHYL]-2-ETHYLBENZYL]AZETIDIN-3-CARBONSÄURE ZUR BEHANDLUNG VON LYMPHOCYTENVERMITTELTEN KRANKHEITEN
SEL HÉMIFUMARATE D'ACIDE 1-[4-[1-(4-CYCLOHEXYL-3-TRIFLUOROMÉTHYL-BENZYLOXYIMINO)-ÉTHYL]-2-ÉTHYL-BENZYL]-AZÉTIDINE-3-CARBOXYLIQUE POUR UNE UTILISATION DANS LE TRAITEMENT DES MALADIES LIEES AUX LYMPHOCYTES

(30) Priority: 18.12.2008 US 203053 P
(43) Date of publication of application: 25.12.2013
(62) Divisional of application: 09793413.7
(73) Proprietor: Novartis AG, 4056 Basel (CH)
(72) Inventor: Ciszewski, Lech, East Hannover, NJ New Jersey 07936 (US); De La Cruz, Marilyn, East Hannover, NJ New Jersey 07936 (US); Karpinski, Piotr H., East Hannover, NJ New Jersey 07936 (US); Mutz, Michael, 4002 Basel (CH); Riegert, Christian, 4002 Basel (CH); Vogel, Caspar, 4002 Basel (CH); Sschneeberger, Ricardo, 4002 Basel (CH)
(74) Representative: Wiessner, Michael

(56) References cited:
- WO-A2-2004/103306

## Description

### Field of the Invention

This invention relates to the hemifumarate salt form of 1-(4-{1-[(E)-4-cyclohexyl-3-trifluoromethyl-benzyloxyimino] -ethyl} -2-ethyl-benzyl)-azetidine-3-carboxylic acid (hereinafter referred to as Compound I) for use in medical treatment and to pharmaceutical compositions comprising this salt form.

### Background of the Invention

It is important to identify forms of a drug that can be conveniently manufactured, formulated and administered to a patient.

Furthermore, in the manufacture of oral drug compositions, it is important that the drug is in a form that provides reliable and reproducible plasma concentrations following administration to a patient.

Chemical stability, solid state stability and "shelf life" of the drug substance are also a particularly important factors. The drug substance, and compositions containing it, should ideally be capable of being effectively stored over appreciable periods of time, without exhibiting a significant change in the active component's physico-chemical characteristics (e.g. its chemical composition, density, hygroscopicity and solubility).

Moreover, it is also important to be able to provide drug in a form which is as chemically pure as possible.

It is known that amorphous drug materials may present some problems in this regard. For example, such materials are typically difficult to handle and to formulate, provide for unreliable solubility, and are often found to be unstable and chemically impure.

The skilled person will therefore appreciate that, if a drug can be readily obtained in a stable crystalline form, many of the above problems may be solved. Thus, in the manufacture of commercially viable, and pharmaceutically acceptable, drug compositions, it is important, wherever possible, to provide drug in a substantially crystalline and stable form. It is to be noted, however, that this goal is not always achievable. Indeed, based on molecular structure alone, it is not typically possible to predict what the crystallisation behaviour of a compound, either as such or in the form of a salt, will be. This can only be determined empirically.

WO2004/103306, the entire contents of which are incorporated herein by reference, discloses a series of compounds capable of inhibiting EDG receptors. WO2004/103306 teaches that the compounds disclosed therein are potentially useful agents for use in the therapy of a number of medical conditions mediated by lymphocytes, such as, for example, transplant rejection, autoimmune conditions and cancer. A full list of possible conditions is recited at page 13, line 9 to page 14, line 3 of WO2004/103306. One particular compound disclosed in WO2004/103306 is 1-(4-{1-[(E)-4-cyclohexyl-3-trifluoromethyl-benzyloxyimino]-ethyl}-2-ethyl-benzyl)-azetidine-3-carboxylic acid (Compound I), the structure of which is shown below. 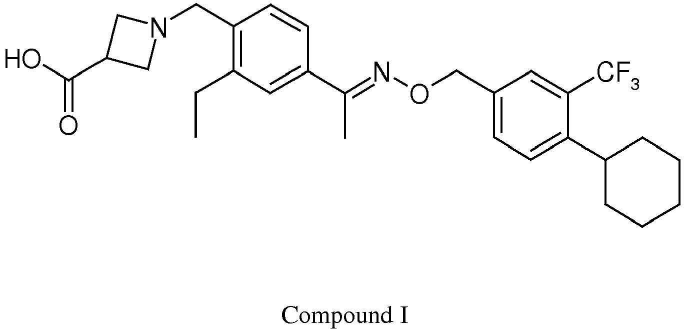 However, there is no disclosure in WO2004/103306 of any salt or crystalline forms of Compound I.

### Summary of the Invention

In a first aspect, there is provided a hemifumarate salt of Compound I for use in treating or preventing various diseases.

In a second aspect, there is provided a pharmaceutical composition comprising the hemifumarate salt of Compound I.

### Summary of Figures

**Figure 1** shows a representative XRPD-diffractogram of Crystalline Form A of the hemifumarate salt of Compound I.
**Figure 2** shows a XRPD-diffractogram of Crystalline Form B of the hemifumarate salt of Compound I.
**Figure 3** shows a representative XRPD-diffractogram of Crystalline Form C of the hemifumarate salt of Compound I.
**Figure 4** shows a XRPD-diffractogram of Crystalline Form D of the hemifumarate salt of Compound I.
**Figure 5** shows a representative FT-Raman spectrum of Crystalline Form A of the hemifumarate salt of Compound I.
**Figure 6** shows a representative FT-Raman spectrum of Crystalline Form B of the hemifumarate salt of Compound I.
**Figure 7** shows a representative FT-Raman spectrum of Crystalline Form C of the hemifumarate salt of Compound I.
**Figure 8** shows a representative FT-Raman spectrum of Crystalline Form D of the hemifumarate salt of Compound I.
**Figure 9** shows a FT-Raman spectrum of Crystalline Form E of the hemifumarate salt of Compound I.

### Disclosure of the Invention

It has now been found that the hemifumarate salt of Compound I is useful in the therapy of diseases or disorders mediated by lymphocyte interactions, such as, for example, in transplantation, such as acute or chronic rejection of cell, tissue or organ allo- or xenografts or delayed graft function, graft versus host disease; rheumatoid arthritis, systemic lupus erythematosus, hashimoto's thyroidis, myasthenia gravis, diabetes type I or II and the disorders associated therewith, vasculitis, pernicious anemia, Sjoegren syndrome, uveitis, psoriasis, Graves ophthalmopathy, alopecia areata and others; allergic diseases, preferably allergic asthma, atopic dermatitis, allergic rhinitis/conjunctivitis, allergic contact dermatitis; inflammatory diseases optionally with underlying aberrant reactions, preferably inflammatory bowel disease, Crohn's disease or ulcerative colitis, intrinsic asthma, inflammatory lung injury, inflammatory liver injury, inflammatory glomerular injury, atherosclerosis, osteoarthritis, irritant contact dermatitis and further eczematous dermatitises, seborrhoeic dermatitis, cutaneous manifestations of immunologically-mediated disorders, inflammatory eye disease, keratoconjunctivitis, inflammatory myopathy; myocarditis or hepatitis; ischemia/reperfusion injury, preferably myocardial infarction, stroke, gut ischemia, renal failure or hemorrhage shock, traumatic shock; T cell lymphomas or T cell leukemias; infectious diseases, preferably toxic shock, septic shock, adult respiratory distress syndrome or viral infections, preferably AIDS, viral hepatitis, chronic bacterial infection; muscle diseases, preferably polymyositis; or senile dementia. Examples of cell, tissue or solid organ transplants include pancreatic islets, stem cells, bone marrow, corneal tissue, neuronal tissue, heart, lung, combined heart-lung, kidney, liver, bowel, pancreas, trachea or oesophagus. For the above uses the required dosage will of course vary depending on the mode of administration, the particular condition to be treated and the effect desired.

The hemifumarate salt of Compound I is useful in the therapy of diseases or disorders mediated by lymphocyte interactions, such as the diseases mentioned above, except for multiple sclerosis.

Furthermore, the hemifumarate salt of Compound I is potentially useful in cancer chemotherapy, particularly for cancer chemotherapy of solid tumors, preferably breast cancer, or as an anti-angiogenic agent.

In addition, the hemifumarate salt of Compound I may be useful in the therapy of a variety of peripheral neuropathies, particularly acute or chronic demyelinating neuropathies. The hemifumarate salt of Compound I may therefore be useful in the therapy of one or more of Guillain-Barré syndrome (GBS), chronic inflammatory demyelinating polyradiculoneuropathy (CIDP), multifocal motor neuropathy with conduction block (MMN), and paraproteinaemic demyelinating peripheral neuropathy (PDN). In particular, the neuropathy is CIPD. The effectiveness of the compounds may vary between patients.

The hemifumarate salt of compound I is potentially useful for treating uveitis, inflammatory bowel disease, Crohn's disease, ulcerative colitis, infectious diseases, chronic inflammatory demyelinating polyradiculoneuropathy (CIDP) and polymyositis in particular.

The therapeutic use of the compound may include prophylactic use to prevent, control or reduce the severity of a peripheral neuropathy which the subject is at risk of suffering, as well as treatment to control or reduce the severity of existing disease. The compound may be administered before the onset of symptoms; it may be administered after the onset of symptoms. It may be administered to a subject at risk of suffering a peripheral neuropathy.

The treatments for which the hemifumarate salt of Compound I may be used may therefore improve, maintain or delay the deterioration of the medical condition and/or comfort of a patient having, suspected of having, or at risk of having, a peripheral neurapathy.

The term "therapy" includes treatment to alleviate one or more symptoms of a peripheral neurapathy or to delay progression of such a disease; it also includes treatment to cure such a disease, to put a subject into a functional state and/or maintain a subject in a functional state, or to prolong time to relapse.

The required dosage will of course vary depending on the mode of administration, the particular condition to be treated and the effect desired. In general, satisfactory results are indicated to be obtained systemically at daily dosages of between about 0.01 to 500 mg per kg patient body weight per day which can be administered in single or multiple doses. The dosage level may be about 0.1 to about 250 mg/kg per day; e.g. about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions may be provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0 or 1000.0 milligrams of the active ingredient. The compounds may be administered on a regimen of 1 to 4 times per day, preferably once or twice per day, more preferably once a day. The dosage regimen may be adjusted to provide the optimal therapeutic response.

In accordance with the foregoing the present invention provides:
1.01 A hemifumarate salt of Compound I for use in preventing or treating disorders or diseases mediated by lymphocytes such as indicated above, in a subject in need of such treatment;
1.02 A hemifumarate salt of Compound I for use in preventing or treating disorders or diseases mediated by lymphocytes, except for multiple sclerosis, in a subject in need of such treatment;
1.03 A hemifumarate salt of Compound I for use in preventing or treating organ or tissue transplant rejection, graft versus host disease, autoimmune diseases, allergic diseases, inflammatory diseases or conditions, or muscle diseases in a subject in need of such treatment;
1.04 A hemifumarate salt of Compound I for use in preventing or treating acute or chronic transplant rejection or T-cell mediated inflammatory or autoimmune diseases, e.g. as indicated above, in a subject in need of such treatment;
1.05 A hemifumarate salt of Compound I for use in inhibiting or controlling deregulated angiogenesis, e.g. sphingosine-1-phosphate (SIP) mediated angiogenesis, in a subject in need thereof;
1.06 A hemifumarate salt of Compound I for use in preventing or treating diseases mediated by a neo-angiogenesis process or associated with deregulated angiogenesis in a subject in need thereof;
1.07 A hemifumarate salt of Compound I for use in preventing or treating cancer in a subject in need thereof;
1.08 A hemifumarate salt of Compound I for use in preventing or treating a peripheral neuropathy in a subject in need thereof;
1.09 A hemifumarate salt of Compound I for use in preventing or treating a peripheral neuropathy selected from Guillain-Barré syndrome, chronic inflammatory demyelinating polyradiculoneuropathy (CIPD), multifocal motor neuropathy with conduction block, and paraproteinaemic demyelinating peripheral neuropathy, in a subject in need thereof;
1.10 A hemifumarate salt of Compound I for use in preventing or treating chronic inflammatory demyelinating polyradiculoneuropathy (CIPD) in a subject in need thereof;
1.11 A hemifumarate salt of Compound I for use in preventing or treating uveitis in a subject in need thereof;
1.12 A hemifumarate salt of Compound I for use in preventing or treating inflammatory bowel disease, Crohn's disease or ulcerative colitits in a subject in need thereof;
1.13 A hemifumarate salt of Compound I for use in preventing or treating inflammatory bowel disease in a subject in need thereof;
1.14 A hemifumarate salt of Compound I for use in preventing or treating Crohn's disease in a subject in need thereof;
1.15 A hemifumarate salt of Compound I for use in preventing or treating ulcerative colitits in a subject in need thereof;
1.16 A hemifumarate salt of Compound I for use in preventing or treating infectious diseases (e.g. bacterial or viral infections) in a subject in need thereof;
1.17 A hemifumarate salt of Compound I for use in preventing or treating viral infections in a subject in need thereof;
1.18 A hemifumarate salt of Compound I for use in preventing or treating polymyositis in a subject in need thereof;
2. A pharmaceutical composition comprising the hemifumarate salt of Compound I for use as defined in paragraphs 1.01 to 1.18 above in association with a pharmaceutically acceptable diluent or carrier therefor.

### Combination therapies

The hemifumarate salt of Compound I may be administered as the sole active ingredient or in conjunction with, e.g. as an adjuvant to, other drugs e.g. immunosuppressive or immunomodulating agents or other anti-inflammatory agents, e.g. for the treatment or prevention of allo- or xenograft acute or chronic rejection or inflammatory or autoimmune disorders, or a chemotherapeutic agent, e.g. a malignant cell anti-proliferative agent. For example the hemifumarate salt of Compound I may be used in combination with a calcineurin inhibitor, preferably cyclosporin A or FK 506; a mTOR inhibitor, preferably rapamycin, 40-0-(2-hydroxyethyl)-rapamycin, CCI779, ABT578 or AP23573; an ascomycin having immunosuppressive properties, preferably ABT-281, ASM981; corticosteroids; cyclophosphamide; azathioprene; methotrexate; leflunomide; mizoribine; mycophenolic acid; mycophenolate mofetil; 15-deoxyspergualine or an immunosuppressive homologue, analogue or derivative thereof; immunosuppressive monoclonal antibodies, preferably monoclonal antibodies to leukocyte receptors, preferably MHC, CD2, CD3, CD4, CD7, CD8, CD25, CD28, CD40. CD45, CD58, CD80, CD86 or their ligands; other immunomodulatory compounds, e.g. a recombinant binding molecule having at least a portion of the extracellular domain of CTLA4 or a mutant thereof, preferably an at least extracellular portion of CTLA4 or a mutant thereof joined to a non-CTLA4 protein sequence, preferably CTLA4Ig or a mutant thereof, preferably LEA29Y; adhesion molecule inhibitors, preferably LF A- I antagonists, ICAM-1 or -3 antagonists, VCAM-4 antagonists or VLA-4 antagonists; or a chemotherapeutic agent.

By the term "chemotherapeutic agent" is meant any chemotherapeutic agent and it includes but is not limited to,
i. an aromatase inhibitor,
ii. an anti-estrogen, an anti-androgen (especially in the case of prostate cancer) or a gonadorelin agonist,
iii. a topoisomerase I inhibitor or a topoisomerase II inhibitor,
iv. a microtubule active agent, an alkylating agent, an antineoplastic antimetabolite or a platin compound,
v. a compound targeting/decreasing a protein or lipid kinase activity or a protein or lipid phosphatase activity, a further anti-angiogenic compound or a compound which induces cell differentiation processes,
vi. a bradykinin I receptor or an angiotensin II antagonist,
vii. a cyclooxygenase inhibitor, a bisphosphonate, a histone deacetylase inhibitor, a heparanase inhibitor (prevents heparan sulphate degradation), preferably PI-88, a biological response modifier, preferably a lymphokine or interferons, preferably interferon γ, an ubiquitination inhibitor, or an inhibitor which blocks anti-apoptotic pathways,
viii. an inhibitor of Ras oncogenic isoforms, preferably H-Ras, K-Ras or N-Ras, or a farnesyl transferase inhibitor, preferably L-744,832 or DK8G557,
ix. a telomerase inhibitor, preferably telomestatin,
x. a protease inhibitor, a matrix metalloproteinase inhibitor, a methionine aminopeptidase inhibitor, preferably bengamide or a derivative thereof, or a proteosome inhibitor, preferably PS-341, and/or
xi. a mTOR inhibitor.

The term "aromatase inhibitor" as used herein relates to a compound which inhibits the estrogen production, i.e. the conversion of the substrates androstenedione and testosterone to estrone and estradiol, respectively. The term includes, but is not limited to steroids, especially atamestane, exemestane and fonnestane and, in particular, non-steroids, especially amino glutethimide, roglethimide, pyridoglutethimide, trilostane, testolactone, ketokonazole, vorozole, fadrozole, anastrozole and letrozole. A combination of the invention comprising a chemotherapeutic agent which is an aromatase inhibitor is particularly useful for the treatment of hormone receptor positive tumors, preferably breast tumors.

The term "anti-estrogen" as used herein relates to a compound which antagonizes the effect of estrogens at the estrogen receptor level. The term includes, but is not limited to tamoxifen, fulvestrant, raloxifene and raloxifene hydrochloride. A combination of the invention comprising a chemotherapeutic agent which is an anti-estrogen is particularly useful for the treatment of estrogen receptor positive tumors, preferably breast tumors.

The term "anti-androgen" as used herein relates to any substance which is capable of inhibiting the biological effects of androgenic hormones and includes, but is not limited to, bicalutamide.

The term "gonadorelin agonist" as used herein includes, but is not limited to abarelix, goserelin and goserelin acetate.

The term "topoisomerase I inhibitor" as used herein includes, but is not limited to topotecan, irinotecan, 9-nitrocamptothecin and the macromolecular camptothecin conjugate PNU-166148 (compound A 1 in WO99/17804).

The term "topoisomerase II inhibitor" as used herein includes, but is not limited to the anthracyclines such as doxorubicin, daunorubicin, epirubicin, idarubicin and nemorubicin, the anthraquinones mitoxantrone and losoxantrone, and the podophillotoxines etoposide and teniposide.

The term "microtubule active agent" relates to microtubule stabilizing and microtubule destabilizing agents including, but not limited to taxanes, preferably paclitaxel and docetaxel, vinca alkaloids, preferably vinblastine, especially vinblastine sulfate, vincristine especially vincristine sulfate, and vinorelbine, discodennolides and epothilones and derivatives thereof, preferably epothilone B or a derivative thereof.

The term "alkylating agent" as used herein includes, but is not limited to busulfan, chlorambucil, cyclophosphamide, ifosfamide, melphalan or nitrosourea (BCNU or Gliadel™).

The term "antineoplastic antimetabolite" includes, but is not limited to 5-fluorouracil, capecitabine, gemcitabine, cytarabine, fludarabine, thioguanine, methotrexate and edatrexate.

The term "platin compound" as used herein includes, but is not limited to carboplatin, cis-platin and oxaliplatin.

The term "compounds targeting/decreasing a protein or lipid kinase activity or further anti-angiogenic compounds" as used herein includes, but is not limited to protein tyrosine kinase and/or serine and/or threonine kinase inhibitors or lipid kinase inhibitors, preferably compounds targeting, decreasing or inhibiting the activity of the epidermal growth factor family of receptor tyrosine kinases (EGFR, ErbB2, ErbB3, ErbB4 as homo- or heterodimers), the vascular endothelial growth factor family of receptor tyrosine kinases (VEGFR), the platelet-derived growth factor-receptors (PDGFR), the fibroblast growth factor-receptors (FGFR), the insulin-like growth factor receptor 1 (IGF-IR), the Trk receptor tyrosine kinase family, the Axl receptor tyrosine kinase family, the Ret receptor tyrosine kinase, the KitlSCFR receptor tyrosine kinase, members of the c-Abl family and their genefusion products, preferably BCR-Abl, members of the protein kinase C (PKC) and Raf family of serine/threonine kinases, members of the MEK, SRC, JAK, FAK, PDK or PI(3) kinase family, or of the PI(3)-kinase-related kinase family, and/or members of the cyclin-dependent kinase family (CDK) and anti-angiogenic compounds having another mechanism for their activity, e.g. unrelated to protein or lipid kinase inhibition.

Compounds which target, decrease or inhibit the activity of VEGFR are especially compounds, proteins or antibodies which inhibit the VEGF receptor tyrosine kinase, inhibit a VEGF receptor or bind to VEGF, and are preferably 1-(4-chloroanilino)-4-(4-pyridylmethyl)phthalazine or a pharmaceutically acceptable salt thereof, e.g. the succinate, a N-aryl(thio) anthranilic acid amide derivative, preferably 2-[(4-pyridyl)methyl]amino-N-[3-methoxy-5-(trifluoromethyl)phenyl]benzamide or 2-[(1-oxido-4-pyridyl)methyl]amino-N-[3-trifluoromethylphenyl]benzamide, Angiostatin^{™}; Endostatin^{™}; anthranilic acid amides; ZD4190; ZD6474; SU5416; SU6668; or anti-VEGF antibodies or anti-VEGF receptor antibodies, preferably RhuMab.

By antibody is meant intact monoclonal antibodies, polyclonal antibodies, multispecific antibodies formed from at least 2 intact antibodies, and antibody fragments so long as they exhibit the desired biological activity.

Compounds which target, decrease or inhibit the activity of the epidermal growth factor receptor family are especially compounds, proteins or antibodies which inhibit members of the EGF receptor tyrosine kinase family, preferably EGF receptor, ErbB2, ErbB3 and ErbB4 or bind to EGF or EGF related ligands, or which have a dual inhibiting effect on the ErbB and VEGF receptor kinase and are preferably trastuzumab (Herpetin®), cetuximab, Iressa, OSI-774, CI-1033, EKB-569, GW-2016, E1.1, E2.4, E2.5, E6.2, E6.4, E2.11, E6.3 or E7.6.3.

Compounds which target, decrease or inhibit the activity of PDGFR are especially compounds which inhibit the PDGF receptor, preferably a N-phenyl-2-pyrimidine-amine derivative, preferably imatinib.

Compounds which target, decrease or inhibit the activity of c-AbI family members and their gene fusion products are, preferably a N-phenyl-2-pyrimidine-amine derivative, e.g. imatinib; PD180970; AG957; or NSC 680410.

Compounds which target, decrease or inhibit the activity of protein kinase C, Raf, MEK, SRC, JAK, FAK and PDK family members, or PI(3) kinase or PI(3) kinase-related family members, and/or members of the cyclin-dependent kinase family (CDK) are staurosporine derivatives, preferably midostaurin; UCN-Ol, safingol, BAY 43-9006, Bryostatin 1, Perifosine; Ilmofosine; RO 318220 and RO 320432; GO 6976; Isis 3521; or LY333531/LY379196.

Further anti-angiogenic compounds are preferably thalidomide (THALOMID) and TNP- 470.

Compounds which target, decrease or inhibit the activity of a protein or lipid phosphatase are, preferably inhibitors of phosphatase 1, phosphatase 2A, PTEN or CDC25, preferably okadaic acid or a derivative thereof.

Compounds which induce cell differentiation processes are, preferably retinoic acid, α-, γ- or δ-tocopherol or α-, γ- or δ-tocotrienol.

The term cyclooxygenase inhibitor as used herein includes, but is not limited to celecoxib (Celebrex^{®}), rofecoxib (Vioxx^{®}), etoricoxib, valdecoxib or a 5-alkyl-2-arylaminophenylacetic acid, preferably 5-methyl-2-(2'-chloro-6'-fluoroanilino)phenyl acetic acid.

The term "histone deacetylase inhibitor" as used herein includes, but is not limited to MS-275, SAHA, pyroxamide, FR-901228 or valproic acid.

The term "bisphosphonates" as used herein includes, but is not limited to, etridonic, clodronic, tiludronic, pamidronic, alendronic, ibandronic, risedronic and zoledronic acid.

The term "matrix metalloproteinase inhibitor" as used herein includes, but is not limited to collagen peptidomimetic and non-petidomimetic inhibitors, tetracycline derivatives, preferably hydroxamate peptidomimetic inhibitor batimastat and its orally bioavailable analogue marimastat, prinomastat, BMS-279251, BAY 12-9566, TAA211 or AAJ996.

The term "mTOR inhibitor" as used herein includes, but is not limited to rapamycin (sirolimus) or a derivative thereof, preferably 32-deoxorapamycin, 16-pent-2-ynyloxy-32-deoxorapamycin, 16-pent-2-ynyloxy-32(S)-dihydro-rapamycin, 16-pent-2-ynyloxy-32(S)-dihydro-40-0-(2-hydroxyethyl)-rapamycin and, more preferably, 40-0-(2-hydroxyethyl)-rapamycin. Further examples of rapamycin derivatives include e.g. CCI779 or 40-[3-hydroxy-2-(hydroxymethyl)-2-methylpropanoate]-rapamycin or a pharmaceutically acceptable salt thereof, ABT578 or 40-(tetrazolyl)-rapamycin, particularly 40-epi-(tetrazolyl)-rapamycin, or rapalogs.

Where the hemifumarate salt of Compound I is administered in conjunction with other immunosuppressive, immunomodulatory, anti-inflammatory or chemotherapeutic therapy, dosages of the co-administered immunosuppressant, immunomodulatory, anti-inflammatory, or chemotherapeutic compound will of course vary depending on the type of co-drug employed, e.g. whether it is a steroid or a calcineurin inhibitor, on the specific drug employed, on the condition being treated and so forth.

For the treatment of peripheral neuropathy the hemifumarate salt of Compound I, may be administered with a further therapeutic agent useful for treating a peripheral neuropathy, for example a demyelinating peripheral neuropathy. By way of example, a second therapeutic agent may be an immunosuppresant (e.g., cyclosporin A, cyclosporin G, FK-506, ABT-281, ASM981, rapamycin, 40-O-(2-hydroxy)ethyl-rapamycin, corticosteroids, cyclophosphamide, azathiopriήe, methotrexate, leflunomide, mizoribine, mycophenolate mofetil, or 15-deoxyspergualine), a steroid (e.g., prednisone or hydrocortisone), an immunoglobulin, or type 1 interferon. The hemifumarate salt of Compound I and the second agent can be administered simultaneously or consecutively.

In accordance with the foregoing the present invention provides in a yet further aspect:
3. A pharmaceutical combination, e.g. a kit, comprising a) a first agent which is a hemifumarate salt of Compound I as disclosed herein, and b) at least one co-agent, e.g. an immunosuppressant, immunomodulatory, anti-inflammatory or chemotherapeutic drug, e.g. as disclosed above.

The kit may comprise instructions for its administration.

The term "pharmaceutical combination" as used herein means a product that results from the mixing or combining of more than one active ingredient and includes both fixed and non-fixed combinations of the active ingredients. The term "fixed combination" means that the active ingredients, e.g. a hemifumarate salt of Compound I and a co-agent, are both administered to a patient simultaneously in the form of a single entity or dosage. The term "non-fixed combination" means that the active ingredients, e.g. a hemifumarate salt of Compound I and a co-agent, are both administered to a patient as separate entities either simultaneously, concurrently or sequentially with no specific time limits, wherein such administration provides therapeutically effective levels of the two compounds in the body of the patient. The latter also applies to cocktail therapy, e.g. the administration of three or more active ingredients.

### The Hemifumarate Salt of Compound I

The hemifumarate salt of Compound I may exist in the amorphous form or it may exist in one or more crystalline forms, as described further below.

Suitably, the hemifumarate salt of Compound I is substantially crystalline. By "substantially crystalline", we mean that the degree of crystallinity, as determined by X-ray powder diffraction data, is conveniently greater than about 20%, more conveniently greater than 60%, even more conveniently greater than about 80%, and preferably greater than about 90%.

The crystalline forms of the present invention can be characterised by X-ray powder diffraction (XRPD). Other techniques, such as FT-Raman spectroscopy, differential scanning calorimetry (DSC) and dynamic vapour sorption may also be used.

### Crystalline Form A of the hemifumarate salt of Compound I

According to a further aspect, the present invention provides a Crystalline Form A. of the hemifumarate salt of compound I.

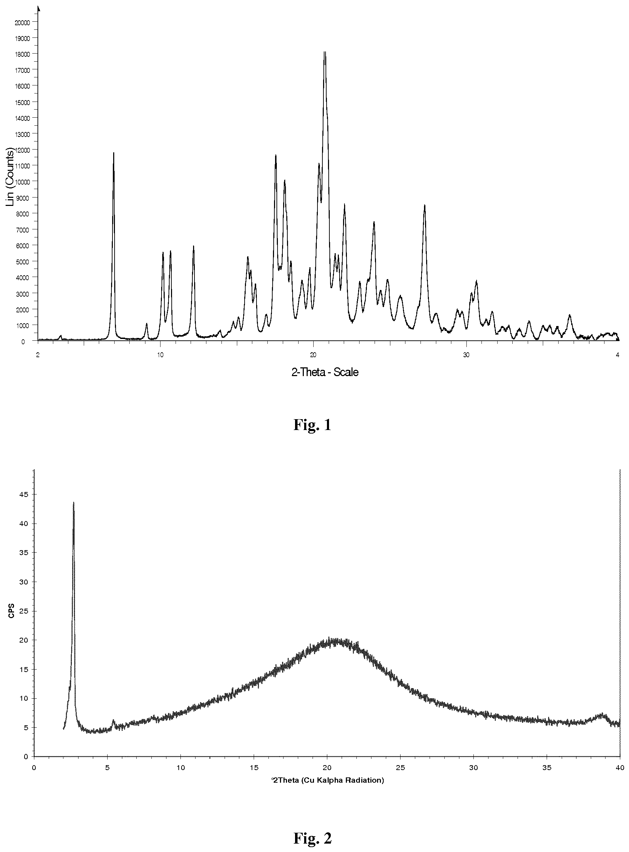

The Crystalline Form A of the hemifumarate salt of Compound I is characterised in that it provides an X-ray powder diffraction pattern substantially as shown in Figure 1.

The most prominent X-ray powder diffraction peaks for the Crystalline Form A of the hemifumarate salt of Compound I are shown in table 1:

**Table 1- The most prominent peaks of Crystalline Form A of the hemifumarate salt of Compound I**

| **2-Theta in deg** | **d value in Angstrom** | **Intensity** |
|---|---|---|
| 6.9 | 12.780 | Medium |
| 10.1 | 8.711 | Medium |
| 10.6 | 8.315 | Medium |
| 12.1 | 7.280 | Medium |
| 15.7 | 5.641 | Medium |
| 16.2 | 5.471 | Small |
| 17.5 | 5.053 | Medium |
| 18.1 | 4.895 | Medium |
| 20.4 | 4.357 | Medium |
| 20.7 | 4.278 | Strong |
| 22.1 | 4.028 | Medium |
| 24.0 | 3.713 | Medium |
| 27.3 | 3.268 | Medium |

According to the present invention there is provided Crystalline Form A of the hemifumarate salt of Compound I, wherein said Crystalline Form has an X-ray powder diffraction pattern with at least one specific peak at about 2-theta = 20.7°.

According to the present invention there is provided Crystalline Form A of the hemifumarate salt of Compound I, wherein said Crystalline Form has an X-ray powder diffraction pattern with at least one specific peak at about 2-theta = 6.9°, 17.5°, 18.1°, or 20.7°.

According to the present invention there is provided Crystalline Form A of the hemifumarate salt of Compound I, wherein said Crystalline Form has an X-ray powder diffraction pattern with specific peaks at about 2-theta = 6.9°, 17.5°, 18.1°, and 20.7°.

According to the present invention there is provided Crystalline Form A of the hemifumarate salt of Compound I, wherein said Crystalline Form has an X-ray powder diffraction pattern with at least one specific peak at about 2-theta = 6.9°, 10.1 °, 10.6 °, 12.1°, 17.5°, 18.1° or 20.7°.

According to the present invention there is provided Crystalline Form A of the hemifumarate salt of Compound I, wherein said Crystalline Form has an X-ray powder diffraction pattern with specific peaks at about 2-theta = 6.9°, 10.1 °, 10.6 °, 12.1°, 17.5°, 18.1° or 20.7°. According to the present invention there is provided Crystalline Form A of the hemifumarate salt of Compound I, wherein said Crystalline Form has an X-ray powder diffraction pattern with specific peaks at about the values listed in Table 1 above.

According to the present invention there is provided Crystalline Form A of the hemifumarate salt of Compound I, wherein said Crystalline Form has an X-ray powder diffraction pattern substantially the same as the X-ray powder diffraction pattern shown in Figure 1.

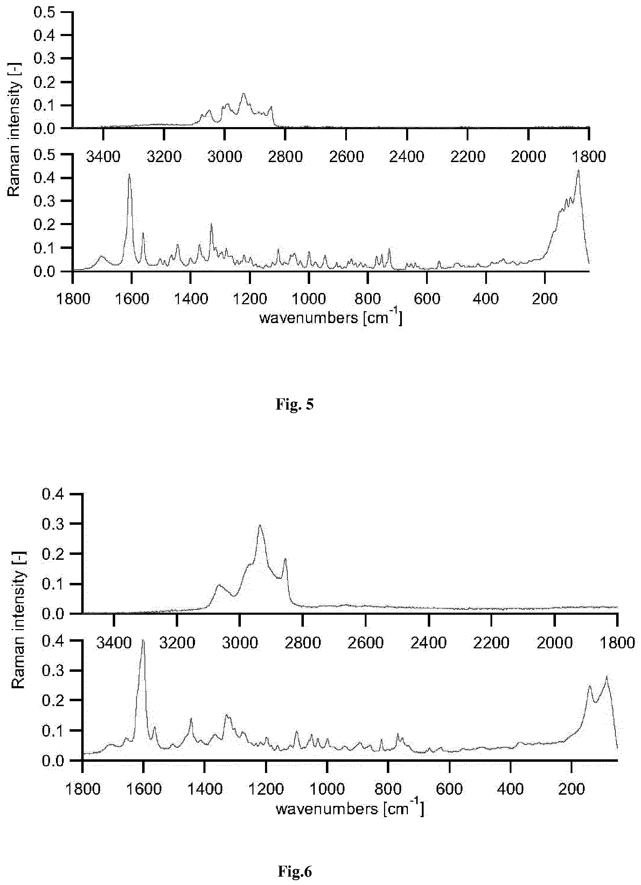

The Crystalline Form A of the hemifumarate salt of Compound I is further characterised by having a FT-Raman spectrum substantially the same as that shown in Figure 5.

Also according to the present invention, there is provided a process for the production of crystalline form A of the hemifumarate salt of compound I, comprising the steps of:
(i) providing a solution containing the hemifumarate salt of compound I; and
(ii) subjecting the solution to conditions of reduced temperature and/or pressure for a time such that formation of crystals of the form A of the hemifumarate salt of compound I takes place.

### Crystalline Form B of the hemifumarate salt of Compound I

According to a further aspect, the present invention provides Crystalline Form B of the hemifumarate salt of compound I.

The Crystalline Form B of the hemifumarate salt of Compound I is characterised in that it provides an X-ray powder diffraction pattern substantially as shown in Figure 2.

Crystalline Form B possesses only a single XRPD peak at 2.7° (2-theta).

Therefore, according to the present invention there is provided Crystalline Form B of the hemifumarate salt of Compound I, wherein said Crystalline Form has an X-ray powder diffraction pattern with a specific peak at about 2-theta = 2.7°.

According to the present invention there is provided Crystalline Form B of the hemifumarate salt of Compound I, wherein said Crystalline Form has an X-ray powder diffraction pattern substantially the same as the X-ray powder diffraction pattern shown in Figure 2.

The Crystalline Form B of the hemifumarate salt of Compound I is further characterised by having a FT-Raman spectrum substantially the same as that shown in Figure 6.

### Crystalline Form C of the hemifumarate salt of Compound I

According to a further aspect, the present invention provides Crystalline Form C of the hemifumarate salt of compound I.

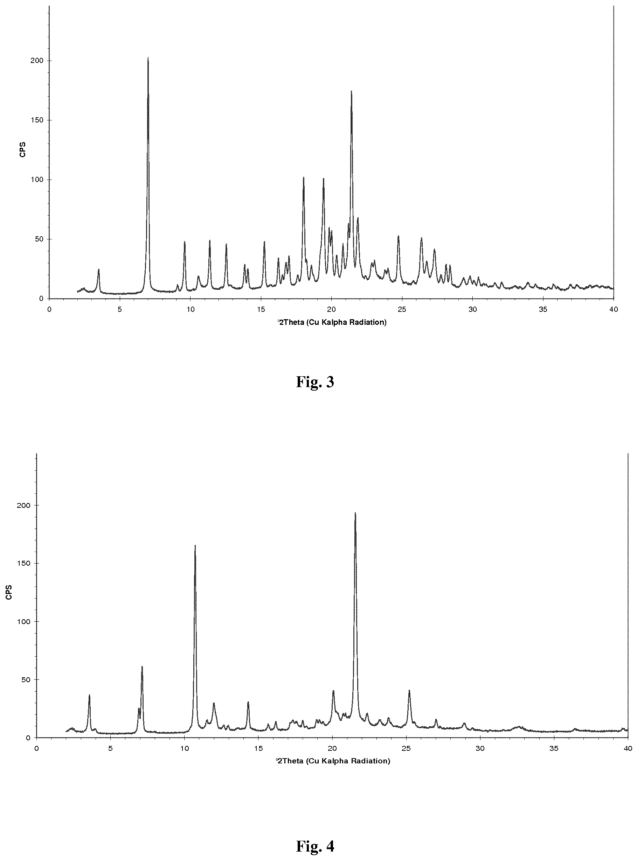

The Crystalline Form C of the hemifumarate salt of Compound I is characterised in that it provides an X-ray powder diffraction pattern substantially as shown in Figure 3.

The most prominent X-ray powder diffraction peaks for the Crystalline Form C of the hemifumarate salt of Compound I are shown in Table 2:

**Table 2 - The most prominent peaks of Crystalline Form C of the hemifumarate salt of Compound I**

| **2-Theta in deg** | **Intensity** |
|---|---|
| 7.0 | strong |
| 9.5 | medium |
| 11.3 | medium |
| 12.5 | medium |
| 15.2 | medium |
| 18.0 | medium |
| 19.4 | medium |
| 21.4 | strong |
| 21.8 | medium |
| 24.7 | medium |

According to the present invention there is provided Crystalline Form C of the hemifumarate salt of Compound I, wherein said Crystalline Form has an X-ray powder diffraction pattern with at least one specific peak at about 2-theta = 7°.

According to the present invention there is provided Crystalline Form C of the hemifumarate salt of Compound I, wherein said Crystalline Form has an X-ray powder diffraction pattern with at least one specific peak at about 2-theta = 21.4 °.

According to the present invention there is provided Crystalline Form C of the hemifumarate salt of Compound I, wherein said Crystalline Form has an X-ray powder diffraction pattern with specific peaks at about 2-theta = 7° and 21.4°.

According to the present invention there is provided Crystalline Form C of the hemifumarate salt of Compound I, wherein said Crystalline Form has an X-ray powder diffraction pattern with specific peaks at about 2-theta = 7°, 9.5 °, 12.5 °, 15.2 ° and 21.4°.

According to the present invention there is provided Crystalline Form C of the hemifumarate salt of Compound I, wherein said Crystalline Form has an X-ray powder diffraction pattern with specific peaks at about the 2-theta values listed in Table 2 above.

According to the present invention there is provided Crystalline Form C of the hemifumarate salt of Compound I, wherein said Crystalline Form has an X-ray powder diffraction pattern substantially the same as the X-ray powder diffraction pattern shown in Figure 3.

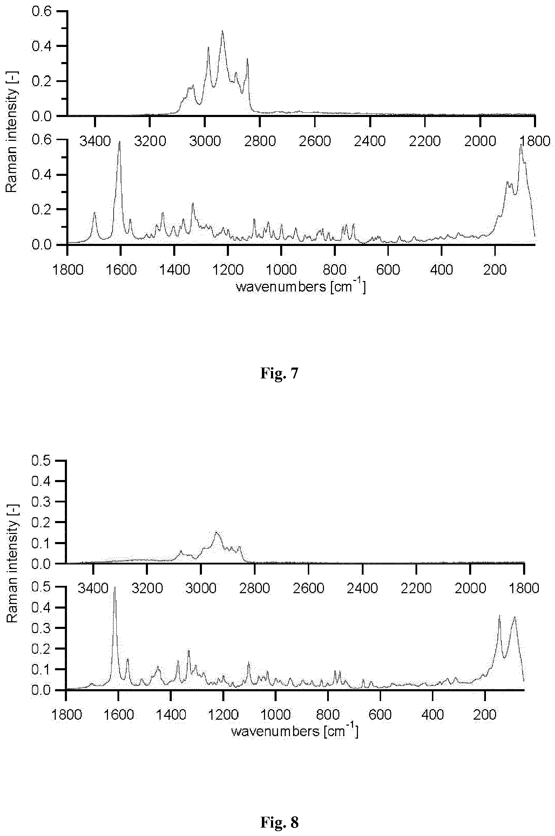

The Crystalline Form C of the hemifumarate salt of Compound I is further characterised by having a FT-Raman spectrum substantially the same as that shown in Figure 7.

Also according to the present invention, there is provided a process for the production of crystalline form C of the hemifumarate salt of compound I, comprising the steps of:
(i) providing a solution containing the hemifumarate salt of compound I; and
(ii) subjecting the solution to conditions of reduced temperature and/or pressure for a time such that formation of crystals of the form C of the hemifumarate salt of compound I takes place.

### Crystalline Form D of the hemifumarate salt of Compound I

According to a further aspect, the present invention provides Crystalline Form D of the hemifumarate salt of compound I.

The Crystalline Form D of the hemifumarate salt of Compound I is characterised in that it provides an X-ray powder diffraction pattern substantially as shown in Figure 4.

The most prominent X-ray powder diffraction peaks for the Crystalline Form D of the hemifumarate salt of Compound I are shown in Table 3:

**Table 3 - The most prominent peaks of Crystalline Form D of the hemifumarate salt of Compound I**

| **2-Theta in deg** | **Intensity** |
|---|---|
| 3.5 | medium |
| 7.1 | medium |
| 10.7 | strong |
| 12.0 | medium |
| 14.3 | medium |
| 20.0 | medium |
| 21.5 | strong |
| 25.2 | medium |

According to the present invention there is provided Crystalline Form D of the hemifumarate salt of Compound I, wherein said Crystalline Form has an X-ray powder diffraction pattern with at least one specific peak at about 2-theta = 10.7°.

According to the present invention there is provided Crystalline Form D of the hemifumarate salt of Compound I, wherein said Crystalline Form has an X-ray powder diffraction pattern with at least one specific peak at about 2-theta = 21.5°.

According to the present invention there is provided Crystalline Form D of the hemifumarate salt of Compound I, wherein said Crystalline Form has an X-ray powder diffraction pattern with specific peaks at about 2-theta = 10.7° and 21.5 °.

According to the present invention there is provided Crystalline Form D of the hemifumarate salt of Compound I, wherein said Crystalline Form has an X-ray powder diffraction pattern with at least one specific peak at about 2-theta = 7.1°, 10.7°, or 21.5°.

According to the present invention there is provided Crystalline Form D of the hemifumarate salt of Compound I, wherein said Crystalline Form has an X-ray powder diffraction pattern with specific peaks at about 2-theta = 7.1°, 10.7° and 21.5°.

According to the present invention there is provided Crystalline Form D of the hemifumarate salt of Compound I, wherein said Crystalline Form has an X-ray powder diffraction pattern with specific peaks at about the values shown in Table 3 above.

According to the present invention there is provided Crystalline Form D of the hemifumarate salt of Compound I, wherein said crystalline Form has an X-ray powder diffraction pattern substantially the same as the X-ray powder diffraction pattern shown in Figure 4.

The Crystalline Form D of the hemifumarate salt of Compound I is further characterised by having a FT-Raman spectrum substantially the same as that shown in Figure 8.

### Crystalline Form E of the hemifumarate salt of Compound I

According to a further aspect, the present invention provides Crystalline Form E of the hemifumarate salt of compound I.

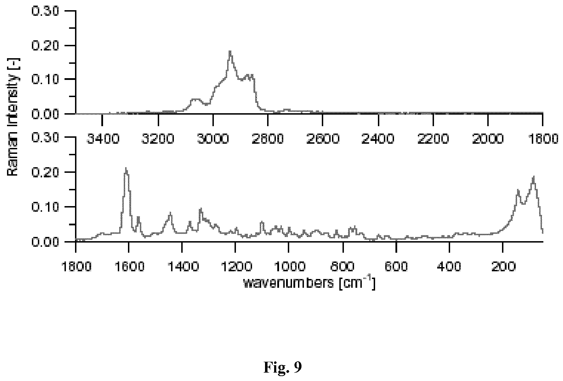

The Crystalline Form E of the hemifumarate salt of Compound I is characterised in that it provides a FT-Raman spectrum substantially the same as that shown in Figure 9.

The crystalline forms of the hemifumarate salt of Compound I, particularly Crystalline Form A, has been found to possess particularly good stability and low hygroscopicity.

The term "stability" as defined herein includes chemical stability and/or solid state stability.

By "chemical stability", we include that the respective compounds can be stored in an isolated form, or in the form of a formulation in which it is provided in admixture with pharmaceutically acceptable carriers, diluents or adjuvants (e.g. in an oral dosage form, such as tablet, capsule etc.), under normal storage conditions, with a limited degree of chemical degradation or decomposition.

By "solid state stability", we include that the respective compounds can be stored in an isolated solid form, or in the form of a solid formulation in which it is provided in admixture with pharmaceutically acceptable carriers, diluents or adjuvants (e.g. in an oral dosage form, such as tablet, capsule etc.), under normal storage conditions, with an insignificant degree of solid state transformation (e.g. crystallisation, recrystallisation, solid state phase transition, hydration, dehydration, solvation or desolvation).

It is known in the art that an X-ray powder diffraction pattern may be obtained which has one or more measurement errors depending on measurement conditions (such as equipment, sample preparation or machine used). In particular, it is generally known that intensities in an X-ray powder diffraction pattern may fluctuate depending on measurement conditions and sample preparation. For example, persons skilled in the art of X-ray powder diffraction will realise that the relative intensity of peaks can be affected by, for example, grains above 30 microns in size and non-unitary aspect ratios, which may affect analysis of samples. The skilled person will also realise that the position of reflections can be affected by the precise height at which the sample sits in the diffractometer and the zero calibration of the diffractometer. The surface planarity of the sample may also have a small effect. Hence a person skilled in the art will appreciate that the diffraction pattern data presented herein is not to be construed as absolute (for further information see Jenkins, R & Snyder, R.L. 'Introduction to X-Ray Powder Diffractometry' John Wiley & Sons, 1996). Therefore, it shall be understood that the crystalline forms of the hemifumarate salt of Compound I of the present invention are not limited to the crystals that provide X-ray powder diffraction patterns identical to the X-ray powder diffraction patterns shown in the accompanying Figures and any crystals providing X-ray powder diffraction patterns substantially the same as that shown in Figures fall within the scope of the present invention. A person skilled in the art of X-ray powder diffraction is able to judge the substantial identity of X-ray powder diffraction patterns.

In the preceding paragraphs defining the X-ray powder diffraction peaks for the crystalline forms of the hemifumarate salt of Compound I, the term "at about" is used in the expression "...at about 2-theta =..." to indicate that the precise position of peaks (i.e. the recited 2-theta angle values) should not be construed as being absolute values. It is also stated in the preceding paragraphs that the crystalline forms of the hemifumarate salt of compound I provide X-ray powder diffraction patterns 'substantially' the same as the X-ray powder diffraction patterns shown in the accompanying figures. It shall be appreciated that the use of the term 'substantially' in this context is also intended to indicate that the 2-theta angle values of the X-ray powder diffraction patterns may vary slightly as consequence of the inherent experimental variations that can occur with these measurements. Consequently, the peaks traces shown in the Figures cannot be construed as absolute.

Generally, a measurement error of a diffraction angle in an X-ray powder diffractogram is about 2-theta = 0.2° or less and such degree of a measurement error should be taken into account when considering the X-ray powder diffraction pattern data described herein. Therefore, where it is stated, for example, that the crystalline form has an X-ray powder diffraction pattern with at least one specific peak at about 2-theta = 15.2° then this can be interpreted as being 2-theta = 15.2° plus or minus 0.2°.

Likewise, the intensity of individual peaks in a FT-Raman spectrum may also change slightly depending on the sample concerned and the measurement conditions, but a person skilled in the art will be able to determine whether two FT-Raman spectra are substantially the same.

### Process of preparation

According to a further aspect of the invention, there is provided a method of preparing a hemifumarate salt of Compound I, said method comprising the step of reacting the free base of Compound I with fumaric acid in the presence of a suitable solvent.

The free base of Compound I can be prepared according to the procedure set out in Example 3 of WO2004/103306.

Any suitable solvent may be used to form the hemifumarate salt of compound I, although the solvent and experimental conditions utilised may influence the solid state form of hemifumarate salt that is obtained.

A person skilled in the art will be able to select appropriate reaction times and conditions for carrying out the salt formation reaction.

Suitably, the free base of compound I is dissolved together with fumaric acid in a suitable solvent (such as those described in the accompanying examples). Alternatively, a solution of free base of Compound I may be dissolved in a suitable solvent and mixed with a solution of fumaric acid (which is dissolved in either the same or a compatible solvent). Suitably, the solution is stirred to facilitate mixing of the free base of Compound I and the fumaric acid. The solution may be mixed at ambient temperature although the procedure may also be performed at higher temperatures.

The hemifumarate salt form of Compound I of the invention may be isolated using techniques which are well known to those skilled in the art, for example decanting, filtering or centrifuging. Suitably, the salt is collected by filtration.

The method may additionally comprise the further steps of washing the hemifumarate salt of Compound I with a suitable solvent; and drying the salt. Preferably the washed salt is dried under vacuum. It will be appreciated by the skilled person that drying temperature and drying time may affect the solid state properties of compounds that are in the form of solvates (e.g. desolvation may occur at elevated temperatures and/or reduced pressure).

The particular crystalline forms A to E of the hemifumarate salt of Compound I can be formed by crystallising the hemifumarate salt from different solvent systems and under different conditions, as described further in the accompanying examples.

In some cases, a particular crystalline form of the hemifumarate salt of Compound I can be formed by reacting the free base of Compound I with fumaric acid under specified conditions. In other cases, a particular crystalline form can be prepared by re-crystallising the hemifumarate salt of Compound I under certain specified conditions.

The invention also provides, a process for preparing crystalline form C of the hemifumarate salt of compound I, comprising the steps of:
(i) providing a solution containing the hemifumarate salt of compound I; and
(ii) subjecting the solution to conditions of reduced temperature and/or pressure for a time such that formation of crystals of the form C of the hemifumarate salt of compound I takes place.

In the above method, the solution of step (i) may be provided by dissolving the hemifumarate salt into a suitable solvent. The selection of a suitable solvent is within the ability of one skilled in the art. An example of a suitable solvent for preparation of the form C of the hemifumarate salt is an alcohol e.g. ethanol.

The solution of step (i) may be provided at an elevated temperature i.e. a temperature above ambient conditions (e.g. 25°C). The elevated temperature is preferably less than 70°C, such as less than 60°C, for example less than 50°C or less than 45°C.

From a temperature solubility profile generated for crystal forms A and C, it is seen that both forms exhibit a similar solubility profile in ethanol at temperatures below 50°C (with form C exhibiting higher solubility at temperatures greater than 50°C). However, surprisingly, it is possible to obtain high yields of Form C (e.g. greater than 60%, 70%, 80%, 90%, 99%) at temperatures less than 50°C using the above method. Without wishing to be bound by theory, it is believed that this surprising result is due to the Form C exhibiting faster precipitation kinetics than form A.

When subjecting the solution to conditions of reduced temperature and/or pressure, the temperature, the pressure or both may be reduced. In preferred embodiments, both the temperature and pressure are reduced, for example by reducing the temperature and then reducing the pressure.

Following the step of subjecting the solution to conditions of reduced temperature and/or pressure, the solution may be subjected to a filtration step in order to recover the crystals of Form C, formed in the process.

In embodiments where the temperature is reduced, the temperature reduction may be greater than 5°C. The temperature reduction may also be less than 30°C, for example less than 20°C or less than 15°C. In an aspect, the temperature reduction is in the range from 8-12°C, e.g. about 10°C.

In embodiments where the pressure is reduced, the pressure may be reduced to a value of less than 200mBar, e.g. less than 100mBar or less than 50mBar. When reduced, the pressure will generally be greater than 10mBar. In an aspect, the pressure is reduced to a value in the range from 10-30mBar e.g. about 20 mBar.

The time required for the formation of crystals of polymorph C in solution during and/or after the step of reducing the temperature and/or pressure of the hemifumarate salt (e.g. before isolation of the crystals from solution e.g. by filtration) will depend on the reaction conditions. In general, to avoid significant loss of the form C crystals once formed (e.g. by conversion to crystals of form A) in solution, the time will be less than 4 hours, e.g. less than 3 hours, such as less than 2 hours or less than 90 minutes e.g. about an hour.

In order to maximise the yield of crystals of form C from solution, the time required for the formation of crystals of polymorph C in solution during and/or after the step of reducing the temperature and/or pressure of the hemifumarate salt (e.g. before isolation of the crystals from solution e.g. by filtration) will generally be greater than 1 minute, for example greater then 5 minutes or greater than 15 minutes such as greater than 30 minutes.

In order to optimise the formation of form C i.e. maximise its formation from solution and minimise its loss due to conversion to other forms, the time may be in the range from 30-90 minutes, e.g. 45-75 minutes e.g. about an hour.

The invention also provides, a process for preparing crystalline form A of the hemifumarate salt of compound I, comprising the steps of:
(i) providing a solvent containing the hemifumarate salt of compound I; and
(ii) subjecting the liquid to conditions of reduced temperature and/or pressure for a time such that formation of crystals of the form A of the hemifumarate salt of compound I takes place.

In the above method, the solution of step (i) may be provided by dissolving the hemifumarate salt into a suitable solvent. The selection of a suitable solvent is within the ability of one skilled in the art. An example of a suitable solvent for preparation of the form A of the hemifumarate salt is an alcohol e.g. ethanol. The solvent may also be a mixture of alcohol (e.g. ethanol) with acid (e.g. fumaric acid) and optionally water e.g. a mixture of ethanol, fumaric acid and water e.g. a 4.35% saturated solution of fumaric acid in a 80%:20% ethanol:water mixture.

The liquid containing the hemifumarate salt of compound I may also be provided by seeding a solvent (e.g. any of the solvents mentioned above) with the hemifumarate salt of compound I in crystal form e.g. to give a solution containing crystals of the hemifumarate salt of compound I e.g. crystals of form A.

The solution of step (i) may be provided at an elevated temperature i.e. a temperature above ambient conditions (e.g. 25°C). The elevated temperature is preferably less than 70°C, such as less than 60°C or less than 55°C. The elevated temperature may also be greater than than 30°C, for example greater than 40°C or 45°C. In an aspect, the elevated temperature is about 50°C.

Following step (i), in cases where the solution contains crystals of the hemifumarate salt of compound I, the solution may be cycled through a temperature cycle during which the solution temperature is raised to a level greater than the elevated temperature (e.g. a level at which greater than 80%, e.g. greater than 90% e.g. greater than 99%, e.g. substantially all of the crystals present in solution are in Form A) and then reduced to a level below the elevated temperature (e.g. to a temperature at which greater than 80%, e.g. greater than 90% e.g. greater than 99%, e.g. substantially all of the crystals present in solution are in Form C) before raising the temperature again to a value above the elevated temperature. This cycling stage ensures that the crystals are predominantly in the required form A before the stage of subjecting the solution to conditions of reduced temperature and/or pressure in stage (ii) above. In addition, the transformation of the crystals from form A to C and back again also serves to reduce the level of impurities within the crystals. The solution may be cycled as described above one or more times e.g. 2 or more; 3 or more or 4 or more times. The solution may be cycled 10 or fewer times, e.g. 8 or fewer times, such as 6 or fewer times. In an aspect, the solution is cycled 1-3 eg. two times.

In an aspect, the temperature is raised above the elevated temperature by about 20°C or less, for example about 15°C or less or about 10°C or less e.g. about 5°C or less. The temperature may also be raised above the elevated temperature by about 1°C or greater, e.g. about 2°C or greater , such as 3°C or greater. In an aspect, the temperature is raised above the elevated temperature by about 3-10°C, e.g. about 4-10°C such as 5-10°C e.g. about 5°C.

In an aspect, the temperature is reduced below the elevated temperature by about 20°C or less, for example about 15°C or less or about 10°C or less e.g. about 5°C or less. The temperature may be reduced below the elevated temperature by about 1°C or greater, e.g. about 2°C or greater , such as 3°C or greater. In an aspect, the temperature is reduced below the elevated temperature by about 3-10°C, e.g. about 4-10°C such as 5-10°C e.g. about 5°C.

During the cycle, the temperature increase may be the same or different from the temperature decrease, e.g. the same. In an aspect, the temperature is raised above the elevated temperature and then lowered below the elevated temperature by about 20°C or less, for example about 15°C or less, or about 10°C or less e.g. about 5°C or less, e.g. about 5°C. Alternatively or in addition, the temperature is raised above the elevated temperature and then lowered below the elevated temperature by about 3-10°C, e.g. about 4-10°C such as 5-10°C e.g. about 5°C.

When subjecting the solution to conditions of reduced temperature and/or pressure, the temperature, the pressure or both may be reduced. In preferred embodiments, both the temperature and pressure are reduced. The temperature may be reduced either simultaneously, sequentially (e.g. by reducing the temperature and then reducing the pressure or vice versa) or in stages (e.g. by reducing the temperature, reducing the pressure and then further reducing the temperature).

In embodiments where the temperature and pressure are reduced in stages, the temperature is preferably initially reduced to a temperature of 25°C or greater before subjecting the solution to conditions of reduced pressure, this initial temperature is also preferably less than 35°C e.g. about 30°C. The solution may then be subjected to conditions of reduced pressure.

Following the step of subjecting the solution to conditions of reduced temperature and/or pressure, the solution may be subjected to a filtration step in order to recover the crystals of Form A, formed in the process.

In embodiments where the temperature is reduced, the total temperature reduction may be greater than 5°C, e.g greater than 10°C, greater than 15°C, greater than 20°C or greater than 25°C. The temperature reduction may also be less than 40°C, e.g. less than 30°C, for example less than 20°C. In an aspect, the temperature reduction is in the range from 35-25°C, e.g. about 30°C.

In order to maximise the amount of crystal form A, the temperature reduction preferably takes place slowly e.g. at a rate of 10°C/hour or less, e.g. 8°C/hour or less, 6°C/hour or less, or 4°C/hour or less. The temperature may also be reduced at a rate greater than 1°C/hour e.g. greater than 2°C/hour.

In embodiments where the pressure is reduced, the pressure may be reduced to a value of less than 300mBar, e.g. less than 200mBar or less than 100mBar. When reduced, the pressure will generally be greater than 30mBar. In an aspect, the pressure is reduced to a value of about 100mBar. The pressure reduction will generally be used to reduce the solvent level e.g. by greater than 5%, e.g. greater than 10%, such as greater than 15%. The solvent level may be reduced by less than 50%, e.g. less than 40% such as less than 30% or less than 20%.

The time required for the formation of crystals of polymorph A in solution during and/or after the step of reducing the temperature and/or pressure of the hemifumarate salt (e.g. before isolation of the crystals from solution e.g. by filtration) will depend on the reaction conditions. In general, to promote formation of crystals of form A, the time may be greater than 2 hours, e.g. greater than 3 hours, e.g. greater than 4 greater or greater than 5 or 6 hours. The time may also be less than 36 hours, e.g. less than 24 hours, e.g. less than 12 or 8 hours.

In a preferred aspect, there is provided a process for preparing crystalline form A of the hemifumarate salt of compound I, comprising the steps of:
(i) providing a solvent containing the hemifumarate salt of compound I, wherein the solution is provided at elevated temperature and contains hemifumarate salt in crystal form; and
(ii) subjecting the liquid to conditions of reduced temperature and/or pressure for a time such that formation of crystals of the form A of the hemifumarate salt of compound I takes place,
wherein prior to step (ii) the solution is cycled through a temperature cycle during which the solution temperature is raised to a level greater than the elevated temperature and then reduced to a level below the elevated temperature before raising the temperature again to a value above the elevated temperature.

In this aspect, the elevated temperature may be about 50°C.

In this aspect, the temperature may be raised above the elevated temperature and then lowered below the elevated temperature by about 3-10°C, e.g. about 4-10°C.

In this aspect, step (ii) the temperature reduction may takes place in stages i.e. the temperature is reduced, the pressure is reduced and then the temperature is reduced again. The temperature may be initially reduced to a value of 25-35°C, e.g. about 30°C before the pressure is reduced. The temperature may then be reduced to about 20-25°C, e.g. about 20°C.

Further experimental details are provided in the Examples.

### Pharmaceutical Preparations

In accordance with the invention, the hemifumarate salt of Compound I (including any one of its crystalline forms A to E as defined herein) may be administered orally, intravenously, subcutaneously, buccally, rectally, dermally, nasally, tracheally, bronchially, by any other parenteral route, or *via* inhalation, in the form of a pharmaceutical preparation comprising the salt or one of its crystalline forms A to E in a pharmaceutically acceptable dosage form.

Typically, therefore, the hemifumarate salt of Compound I (including any one of its crystalline forms A to E) of the invention may be administered orally or parenterally ("parenterally" as used herein, refers to modes of administration which include intravenous, intramuscular, intraperitoneal, intrasternal, subcutaneous and intraarticular injection and infusion) to a host. In the case of larger animals, such as humans, the hemifumarate salt of Compound I (including any one of its crystalline forms A to E) may be administered alone as an alternative to administration as compositions in combination with pharmaceutically acceptable diluents, excipients or carriers.

Depending on the disorder, and the patient to be treated, as well as the route of administration, the hemifumarate salt of Compound I (including any one of its crystalline forms A to E) may be administered at varying doses (see below).

The hemifumarate salt of Compound I (including any one of its crystalline forms A to E) may be further processed before formulation into a suitable pharmaceutical formulation, for example they may be milled or ground into smaller particles.

According to a further aspect of the invention, there is provided a pharmaceutical composition comprising the hemifumarate salt of Compound I (including any one of its crystalline forms A to E) as defined herein in admixture with a pharmaceutically acceptable adjuvant, diluent or carrier.

The amount of the hemifumarate salt of Compound I of the invention which is employed in such a composition will depend on the condition, and patient, to be treated, as well as the crystalline form(s) which is/are employed, but this can be determined non-inventively.

Pharmaceutical compositions of this invention for parenteral injection suitably comprise pharmaceutically acceptable sterile aqueous or non-aqueous solutions, dispersions, suspensions or emulsions as well as sterile powders for reconstitution into sterile injectable solutions or dispersions just prior to use. Examples of suitable aqueous and non-aqueous carriers, diluents, solvents or vehicles include water, ethanol, polyols (such as glycerol, propylene glycol, polyethylene glycol and the like), and suitable mixtures thereof, vegetable oils (such as olive oil) and injectable organic esters such as ethyl oleate. Proper fluidity can be maintained, for example, by the use of coating materials such as lecithin, by the maintenance of the required particle size in the case of dispersions and by the use of surfactants.

These compositions may also contain adjuvants such as preservative, wetting agents, emulsifying agents and dispersing agents. Prevention of the action of microorganisms may be ensured by the inclusion of various antibacterial and antifungal agents, for example, paraben, chlorobutanol or phenol sorbic acid. It may also be desirable to include isotonic agents such as sugars or sodium chloride, for example. Prolonged absorption of the injectable pharmaceutical form may be brought about by the inclusion of agents (for example aluminum monostearate and gelatin) which delay absorption.

In some cases, in order to prolong the effect of the drug, it is desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are suitably made by forming microencapsule matrices of the drug in biodegradable polymers, for example polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations may also prepared by entrapping the drug in liposomes or microemulsions which are compatible with body tissues. The injectable formulations can be sterilized, for example, by filtration through a bacterial-retaining filter or by incorporating sterilizing agents in the form of sterile solid compositions which can be dissolved or dispersed in sterile water or other sterile injectable media just prior to use.

Solid dosage forms for oral administration include capsules, tablets, pills, powders and granules. In such solid dosage forms, the hemifumarate salt of Compound I (including any one of its crystalline forms A to E) is typically mixed with at least one inert, pharmaceutically acceptable excipient or carrier such as sodium citrate or dicalcium phosphate and/or one or more: a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycol, for example.

Suitably, oral formulations contain a dissolution aid. The dissolution aid is not limited as to its identity so long as it is pharmaceutically acceptable. Examples include nonionic surface active agents, such as sucrose fatty acid esters, glycerol fatty acid esters, sorbitan fatty acid esters (e.g. sorbitan trioleate), polyethylene glycol, polyoxyethylene hydrogenated castor oil, polyoxyethylene sorbitan fatty acid esters, polyoxyethylene alkyl ethers, methoxypolyoxyethylene alkyl ethers, polyoxyethylene alkylphenyl ethers, polyethylene glycol fatty acid esters, polyoxyethylene alkylamines, polyoxyethylene alkyl thioethers, polyoxyethylene polyoxypropylene copolymers, polyoxyethylene glycerol fatty acid esters, pentaerythritol fatty acid esters, propylene glycol monofatty acid esters, polyoxyethylene propylene glycol monofatty acid esters, polyoxyethylene sorbitol fatty acid esters, fatty acid alkylolamides, and alkylamine oxides; bile acid and salts thereof (e.g. chenodeoxycholic acid, cholic acid, deoxycholic acid, dehydrocholic acid and salts thereof, and glycine or taurine conjugate thereof); ionic surface active agents, such as sodium laurylsulfate, fatty acid soaps, alkylsulfonates, alkylphosphates, ether phosphates, fatty acid salts of basic amino acids; triethanolamine soap, and alkyl quaternary ammonium salts; and amphoteric surface active agents, such as betaines and aminocarboxylic acid salts.

The solid dosage forms of tablets, dragees, capsules, pills, and granules can be prepared with coatings and shells such as enteric coatings and other coatings well known in the pharmaceutical formulating art. They may optionally contain opacifying agents and may also be of a composition such that they release the active ingredient(s) only, or preferentially, in a certain part of the intestinal tract, and/or in delayed fashion. Examples of embedding compositions include polymeric substances and waxes.

The hemifumarate salt of Compound I (including any one of its crystalline forms A to E) may also be in micro-encapsulated form, if appropriate, with one or more of the above-mentioned excipients.

The hemifumarate salt of Compound I (including any one of its crystalline forms A to E) may be in finely divided form, for example it may be micronised.

Liquid dosage forms for oral administration include pharmaceutically acceptable emulsions, solutions, suspensions, syrups and elixirs. In addition to the hemifumarate salt of Compound I (including any one of its crystalline forms A to E), the liquid dosage forms may contain inert diluents commonly used in the art such as water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan and mixtures thereof. Besides inert diluents, the oral compositions may also include adjuvants such as wetting agents, emulsifying and suspending agents, sweetening, flavoring and perfuming agents. Suspensions, in addition to the active compounds, may contain suspending agents such as ethoxylated isostearyl alcohols, polyoxyethylene sorbitol and sorbitan esters, microcrystalline cellulose, aluminum metahydroxide, bentonite, agar-agar, and tragacanth and mixtures thereof.

Compositions for rectal or vaginal administration are preferably suppositories which can be prepared by mixing the hemifumarate salt of Compound I (including any one of its crystalline forms A to E) of this invention with suitable non-irritating excipients or carriers such as cocoa butter, polyethylene glycol or a suppository wax which are solid at room temperature but liquid at body temperature and therefore melt in the rectum or vaginal cavity and release the active compound.

The hemifumarate salt of Compound I (including any one of its crystalline forms A to E) can also be administered in the form of liposomes. As is known in the art, liposomes are generally derived from phospholipids or other lipid substances. Liposomes are formed by mono- or multi-lamellar hydrated liquid crystals which are dispersed in an aqueous medium. Any non-toxic, physiologically acceptable and metabolisable lipid capable of forming liposomes can be used. The present compositions in liposome form can contain, in addition to a compound of the present invention, stabilisers, preservatives, excipients and the like. The preferred lipids are the phospholipids and the phosphatidyl cholines (lecithins), both natural and synthetic. Methods to form liposomes are known in the art, for example, Prescott, Ed., Methods in Cell Biology, Volume XIV, Academic Press, New York, N.Y. (1976), p 33 et seq.

Advantageously, the hemifumarate salt of Compound I (including any one of its crystalline forms A to E) of the invention may be orally active, have rapid onset of activity and low toxicity.

The actual dosage levels of the hemifumarate salt of Compound I in the pharmaceutical compositions of this invention may be varied so as to obtain an amount of the active drug that is effective to achieve the desired therapeutic response for a particular patient, compositions, and mode of administration. The selected dosage level will depend upon the activity of the compound, the route of administration, the severity of the condition being treated and the condition and prior medical history of the patient being treated. However, it is within the skill of the art to start doses of the compound at levels lower than required in order to achieve the desired therapeutic effect and to gradually increase the dosage until the desired effect is achieved.

### Examples

The invention is illustrated, but in no way limited, by the following Examples and with reference to the enclosed Figures.

### General Procedures

### X-Ray Powder Diffraction

X-ray powder diffraction analysis (XRPD) was performed on samples prepared according to standard methods, for example those described in Giacovazzo, C. et al (1995), Fundamentals of Crystallography, Oxford University Press; Jenkins, R. and Snyder, R. L. (1996), Introduction to X-Ray Powder Diffractometry, John Wiley & Sons, New York; Bunn, C. W. (1948), Chemical Crystallography, Clarendon Press, London; or Klug, H. P. & Alexander, L. E. (1974), X-ray Diffraction Procedures, John Wiley and Sons, New York. X-ray analyses were performed using a Bruker D8 Advance Powder X-ray Diffactometer. Samples were analysed as powder and placed as powder on the specimen holder.

XRPD diffraction angles (2-theta) may vary in the range ±0.2° (2-theta).

In some of the following Examples, the same crystalline form is prepared by different processes. In such cases, reference is made to the same representative characterising data because each process produced the same crystalline form having "essentially" the same XRPD diffraction pattern. In other words, it was clear from the relevant patterns (allowing for experimental error) that the same crystalline form had been prepared.

### Differential Scanning Calorimetry

Differential scanning calorimetry (DSC) was performed using a Perkin Elmer DSC7 instrument, according to standard methods, for example those described in Höhne, G. W. H. et al (1996), Differential Scanning Calorimetry, Springer, Berlin.

### Dynamic Vapour Sorption

Dynamic Vapour Sorption measurements were taken using a DVS-1 water vapour sorption analyzer (Surface Measurement Systems Ltd.) or a Projekt Messtechnik SPS11-100n. The sample was allowed to equilibrate at 50% r.h. before starting a pre-defined humidity program (50-0-95-50% r.h., scanning with 5% Δr.h. hour-1 and with several isohumid equilibration periods).

### Fourier Transform Raman Spectroscopy

FT-Raman spectroscopy measurements are performed on a Bruker RFS100.

### Preparation of starting materials

Unless otherwise described herein, the free base form of Compound I may be prepared as described in Example 3 of WO2004/103306.

### Abbreviations

- EtOH: ethanol
- NaBH(OAc)₃: sodium triacetoxyborohydride
- NaOH: sodium hydroxide
- MEK: 2-butanone
- 2PrOH: 2-propanaol
- THF: tetrahydrofuran

### Example 1 - Preparation of the hemifumarate salt of 1-(4-{1-[(E)-4-Cyclohexyl-3-trifluoromethyl-benzyloxyimino]-ethyl}-2-ethyl-benzyl)-azetidine-3-carboxylic acid (Compound I)

30g of compound I was prepared using the method of Example 3 of WO2004/103306.

The hemifumarate salt of the invention was then prepared by reacting a suspension of 30.0 g of compound I and 20 g fumaric acid (5 % conc.) in 200g ethanol absolute (>99.9 %) at room temperature (25°C).
The stability of the hemifumarate salt solution relative to the free base solution was then tested by subjecting both solutions to conditions of elevated temperature (40°C, 50°C and 60°C) for a period of one week.
The hemifumarate salt exhibited superior stability under all conditions tested.

### Example 2 - Preparation of the Crystalline Form A of the hemifumarate salt of 1-(4-{1-[(E)-4-Cyclohexyl-3-trifluoromethyl-benzyloxyimino]-ethyl}-2-ethyl-benzyl)-azetidine-3-carboxylic acid (Compound I)

### Method 1

4-[1-(4-cyclohexyl-3-trifluoromethyl-benzyloxyimino)-ethyl]-2-ethyl-benzaldehyde (4.32g) and azetidine-3-carboxylic acid (1.42g) were suspended in 75ml of methanol and stirred at a temperature of 23 - 27°C for 15 - 30 minutes. NaBH(OAc)₃ (3.81g) was added in portions over a period of 1-2 hours at 23 - 27°C. Once the reaction was complete, the methanol was distilled off. A further 50 mL of ethyl acetate was added and then distilled off. In the next step, 50 mL of ethyl acetate, 2.55 mL methanol and 25 mL water were added to the distillation residue and the mixture was stirred until two clear phases were obtained. The pH was adjusted to pH 6 by the addition of 2N NaOH and the phases were separated. The organic phase was extracted with 10ml water and concentrated to 50% of the original volume. Absolute ethanol was then added to restore the original volume. These concentration/distillation and addition of absolute ethanol steps were repeated twice. Charcoal (0.43g) and Cellflock (0.43g) were then added and the mixture was stirred for 30 minutes at a temperature of 25°C and then filtered. The filtrate was then concentrated to about 50g and 0.87g fumaric acid was added as a solid at a temperature of 45°C. When everything had dissolved, the solution was seeded with 60mg of 1-(4-{1-[(E)-4-Cyclohexyl-3-trifluoromethyl-benzyloxyimino]-ethyl}-2-ethyl-benzyl)-azetidine-3-carboxylic acid, (E)-but-2-enedioic acid. The crystallization was completed by stirring for 1 hour at a temperature of 40 -45°C, followed by cooling to 20 - 25 °C within 1-2 hours and stirring at 20 - 25°C for a further 15 - 20 hours. The resultant product was collected by filtration and washed to yield 4.9g 1-(4-{1-[(E)-4-Cyclohexyl-3-trifluoromethyl-benzyloxyimino]-ethyl}-2-ethyl-benzyl)-azetidine-3-carboxylic acid, (E)-but-2-enedioic acid as a white powder.

### Method 2

The free base of Compound I (400 mg) and fumaric acid (89 mg) were added into 2 mL EtOH. The mixture was heated to 50°C for 2 hours and then cooled to room temperature with constant stirring. It was then kept cold in a refrigerator for 72 hours and then vacuum filtered to collect the white solids.

### Method 3

The free base of Compound I (100 mg) and 1.5 mL of 0.125mM fumaric acid were added to 1 mL EtOH. The solution was heated to 40°C and allowed to evaporate to dryness. Acetonitrile (2 mL) was added and the mixture was stirred. The white solids were collected by vacuum filtration using qualitative filter paper.

### Method 4

The free base of Compound I (100 mg) and fumaric acid (22.4 mg) were added to 2 mL acetonitrile. The solution was stirred overnight and the mixture was filtered to collect the white solid precipitate.

### Method 5 - Slurry method

The free base of Compound I (400 mg) and fumaric acid (89mg) were added to 10mL acetonitrile. The solution was sonicated and heated to 40°C for 30 minutes and then cooled to room temperature. The solution was stirred for a further 2 hours with addition of 2 mL more of acetonitrile. The solution was then transferred to a refrigerator for 72 hours and the white solids were collected by vacuum filtration.

### Method 6

Crystalline Form A of the hemifumarate salt of Compound I (33.9 mg) was dissolved in 3 ml MEK at 70°C. The clear, slightly yellowish solution was then stored directly in the fridge. After 2 days of storage in the fridge, the white precipitate was collected by filtration over a P4 glass filter and air was sucked through the sample for about 3 minutes.

### Method 7

Crystalline Form A of the hemifumarate salt of Compound I (34.6 mg) was dissolved in 5ml of acetone at 50°C and the resultant clear colourless solution was directly stored in the fridge. After 1 day of storage the white precipitate was filtered over a P4 glass filter and air was sucked through the sample for about 3 minutes.

### Method 8

Fumaric acid present as a 4.35% saturated solution in an 80:20 ethanol:water mixture is added to a reaction vessel as a 4.35 % saturated solution at an inlet temperature of 50°C in two portions. First 10 % of the fumaric acid is added, then the solution is seeded with crystalline form A (obtainable by methods 1-7 above) at an inlet temperature of 50°C, after that the second portion of fumaric acid (90% amount) is added over 2 hours at an inlet temperature of 50°C.

The white precipitate suspension is then heated to 55 °C and cooled to 45°C twice in order to stabilize the Polymorph form A.

Then the crystallisation is completed by slow cooling to a Jacket Temperature of 30°C over 5 hours. The suspension is then reduced by distillation at a Jacket Temperature of 30°C and pressure of 100 mbar to remove 20 % of the suspension amount over 2 hours, and then cooled at an internal temperature of 20°C for 1 hour. Finally, Form A of the hemifumarate salt is isolated by filtration and washed with ALANP before drying at Jacket temperature of 40°C under vacuum overnight.

### Analysis of Crystalline Form A:

XRPD analysis indicated that the products of Method 1 to 8 above was Crystalline Form A of the hemifumarate salt of 1-(4-{1-[(E)-4-Cyclohexyl-3-trifluoromethyl-benzyloxyimino]-ethyl}-2-ethyl-benzyl)-azetidine-3-carboxylic acid (Compound I), having the peaks listed in Table 1 above and an XRPD trace which is the same, or essentially the same, as the representative trace shown in Figure 1. Crystalline Form A produced by Methods 1 to 8 also provides a FT-Raman spectrum that is the same, or essentially the same, as the representative trace shown in Figure 5.

Dynamic Vapour Sorption measurements indicated that Crystalline Form A has a low hygroscopicity.

Crystalline form A was also tested for pressure stability by exposing a few milligrams of this form to a pressure of 10 tons for 5 minutes. The initial and resulting samples were analysed under a Raman microscope, no change in form was observed.

### Example 3 - preparation of the Crystalline Form B of the hemifumarate salt of 1-(4-{1-[(E)-4-Cyclohexyl-3-trifluoromethyl-benzyloxyimino]-ethyl}-2-ethyl-benzyl)-azetidine-3-carboxylic acid (Compound I)

49.7 mg of crystalline Form A of the hemifumarate salt of Compound I was dissolved in 10 ml EtOH and the clear colourless solution was filtered through a 0.2µm PTFE filter and allowed to evaporate at ambient conditions from a crystallization dish of 9 cm diameter. After 2 days a colourless residue was observed and was scratched out of the dish. The resultant fine white powder was obtained and analysed.

### Analysis of Crystalline Form B:

XRPD analysis indicated that the product was Crystalline Form B of the hemifumarate salt of 1-(4-{1-[(E)-4-Cyclohexyl-3-trifluoromethyl-benzyloxyimino]-ethyl}-2-ethyl-benzyl)-azetidine-3-carboxylic acid (Compound I), having a single peak at 2.7° (2-theta) and an XRPD trace as shown in Figure 2. Crystalline Form B was also found to have a FT-Raman spectrum as shown in Figure 6.

### Example 4 - preparation of the Crystalline Form C of the hemifumarate salt of 1-(4-{1-[(E)-4-Cyclohexyl-3-trifluoromethyl-benzyloxyimino]-ethyl}-2-ethyl-benzyl)-azetidine-3-carboxylic acid (Compound I)

### Method 1

103.9 mg of Crystalline Form A of the hemifumarate salt of Compound I was dissolved in 2 ml acetone / water 1:1 at 70°C and the clear colourless solution was directly stored in the fridge. After 1 day of storage the white precipitate was filtered over a P4 glass filter and air was sucked through the sample for about 3 minutes.

### Method 2

35.0 mg of Crystalline Form A of the hemifumarate salt of Compound I was dissolved in 1 ml 2PrOH at 75°C and the clear colourless solution formed was directly stored in the fridge. After 1 day of storage the white precipitate was filtered over a P4 glass filter and air was sucked through the sample for about 3 minutes. The resultant fine white powder was analyzed by FT-Raman spectroscopy and XRPD.

### Method 3

A suspension of 30.0 g solution compound I and 20 g fumaric acid (5 % conc.) in 200 g ethanol absolute (>99.9 %) is heated to 40°C Jacket temperature. The temperature of 40°C is maintained for 1 hour at 200 rpm stirrer speed. Afterwards the solution is cooled to 30 °C and the ethanol solution is distilled at 20 mbar until the solution precipitates. The crystals are collected by directly filtering the suspension, then dried for 8 h at 40 °C and 10 mbar.
A yield of 80% is obtained (i.e. 5.5 g)

### Analysis of Crystalline Form C:

XRPD analysis conducted on the white solids obtained by Methods 1 and 2 above indicated that the product was Crystalline Form C of the hemifumarate salt of 1-(4-{1-[(E)-4-Cyclohexyl-3-trifluoromethyl-benzyloxyimino]-ethyl}-2-ethyl-benzyl)-azetidine-3-carboxylic acid (Compound I), having the peaks listed in Table 2 above and an XRPD trace the same, or substantially the same, as the representative trace shown in Figure 3. Crystalline Form C was also found to have a FT-Raman spectrum the same, or substantially the same, as the representative trace shown in Figure 7.

Dynamic Vapour Sorption measurements indicated that Crystalline Form C was not hygroscopic.

### Example 5 - preparation of the Crystalline Form D of the hemifumarate salt of 1-(4-{1-[(E)-4-Cyclohexyl-3-trifluoromethyl-benzyloxyimino]-ethyl}-2-ethyl-benzyl)-azetidine-3-carboxylic acid (Compound I)

100.6 mg of Crystalline Form A of the hemifumarate salt of Compound I was dissolved in 4.5 ml THF and a clear and colourless solution was obtained. Water was added as antisolvent (5.1 ml:8.2 ml) and a type of emulsion was obtained. The mixture was stirred for 20 minutes and 2.5 ml water was added and the solution was stored in the fridge. After 2 days white precipitate had formed that was isolated over a P4 glass filter. The solution did not well filter. Air was sucked for about 2 minutes through the sample and the white solid was analyzed by FT-Raman spectroscopy and XRPD. After 6 days the sample was dried for a further 15 minutes under vacuum and analyzed again by FT-Raman spectroscopy and XRPD.

### Analysis of Crystalline Form D:

XRPD analysis conducted on the white precipitate indicated that the product was Crystalline Form D of the hemifumarate salt of 1-(4-{1-[(E)-4-Cyclohexyl-3-trifluoromethyl-benzyloxyimino] -methyl}-2-ethyl-benzyl)-azetidine-3-carboxylic acid (Compound I), having the peaks listed in Table 2 above and an XRPD trace as shown in Figure 4. Crystalline Form D was also found to have a FT-Raman spectrum which is the same or essentially the same as the representative trace shown in Figure 8.

### Example 6 - preparation of the Crystalline Form E of the hemifumarate salt of 1-(4-{1-[(E)-4-Cyclohexyl-3-trifluoromethyl-benzyloxyimino]-ethyl}-2-ethyl-benzyl)-azetidine-3-carboxylic acid (Compound I)

100.8 mg of Crystalline Form A of the hemifumarate salt of Compound I was suspended in 1 ml THF / water 1:1 and filtered over a 0.2 µm PTFE filter and a clear and slightly yellowish solution was obtained. 1 ml n-hexane was added as antisolvent and a white precipitate appeared. The mixture was then stirred for a further 5 minutes and then filtered through a 0.45µm PTFE centrifuge filter and the solid all passed the filter. The suspension was re-filtered over a 0.45µm PTFE centrifuge filter for a shorter time and the supernatant was decanted to allow for isolation of the white powder. The wet cake was analysed by FT-Raman spectroscopy and showed the pattern of crystalline Form B of the hemifumarate salt of Compound I.

The solid was then was allowed to dry under ambient conditions in the FT-Raman sample holder. The sample was subsequently analysed by FT-Raman spectroscopy and showed the spectrum of Crystalline Form E, which is shown in Figure 9.

## Claims

1. A hemifumarate salt of 1-(4-{1-[(E)-4-cyclohexyl-3-trifluoromethyl-benzyloxyimino]-ethyl}-2-ethyl-benzyl)-azetidine-3-carboxylic acid for use in treating or preventing diseases or disorders mediated by lymphocyte interactions, wherein the disease or disorder is selected from acute or chronic rejection of cell, tissue or organ allo- or xenografts or delayed graft function, graft versus host disease; rheumatoid arthritis, systemic lupus erythematosus, hashimoto's thyroidis, myasthenia gravis, diabetes type I or II and the disorders associated therewith, vasculitis, pernicious anemia, Sjoegren syndrome, uveitis, psoriasis, Graves ophthalmopathy, alopecia areata; allergic diseases, preferably allergic asthma, atopic dermatitis, allergic rhinitis/conjunctivitis, allergic contact dermatitis; inflammatory diseases optionally with underlying aberrant reactions, preferably inflammatory bowel disease, Crohn's disease or ulcerative colitis, intrinsic asthma, inflammatory lung injury, inflammatory liver injury, inflammatory glomerular injury, atherosclerosis, osteoarthritis, irritant contact dermatitis and further eczematous dermatitises, seborrhoeic dermatitis, cutaneous manifestations of immunologically-mediated disorders, inflammatory eye disease, keratoconjunctivitis, inflammatory myopathy; myocarditis or hepatitis; ischemia/reperfusion injury, preferably myocardial infarction, stroke, gut ischemia, renal failure or hemorrhage shock, traumatic shock; T cell lymphomas or T cell leukemias; infectious diseases, preferably toxic shock, septic shock, adult respiratory distress syndrome or viral infections, preferably AIDS, viral hepatitis, chronic bacterial infection; muscle diseases, preferably polymyositis; or senile dementia; cancer, preferably breast cancer, or as an anti-angiogenic agent; peripheral neuropathies, particularly acute or chronic demyelinating neuropathies such as Guillain-Barré syndrome (GBS), chronic inflammatory demyelinating polyradiculoneuropathy (CIDP), multifocal motor neuropathy with conduction block (MMN), and paraproteinaemic demyelinating peripheral neuropathy (PDN).

2. A hemifumarate salt of 1-(4-{1-[(E)-4-cyclohexyl-3-trifluoromethyl-benzyloxyimino]-ethyl}-2-ethyl-benzyl)-azetidine-3-carboxylic acid for use in treating or preventing diseases or disorders mediated by lymphocyte interactions according to claim 1, wherein the disease or disorder is polymyositis.

3. A hemifumarate salt of 1-(4-{1-[(E)-4-cyclohexyl-3-trifluoromethyl-benzyloxyimino]-ethyl}-2-ethyl-benzyl)-azetidine-3-carboxylic acid for use according to claim 1 to 2, wherein said salt has a degree of crystallinity greater than about 20%, preferably greater than about 90%.

4. A hemifumarate salt of 1-(4-{1-[(E)-4-cyclohexyl-3-trifluoromethyl-benzyloxyimino]-ethyl}-2-ethyl-benzyl)-azetidine-3-carboxylic acid for use according to any of claims 1 to 3, wherein said salt comprises a Crystalline Form A, wherein the Crystalline Form A has an X-ray powder diffraction pattern with at least one specific peak at about 2-theta = 6.9°, 10.1 °, 10.6 °, 12.1°, 17.5°, 18.1° or 20.7°.

5. A hemifumarate salt of 1-(4-{1-[(E)-4-cyclohexyl-3-trifluoromethyl-benzyloxyimino]-ethyl}-2-ethyl-benzyl)-azetidine-3-carboxylic acid for use according to any of claims 1 to 4 wherein the salt comprises a Crystalline Form A, wherein the Crystalline Form A has the following FT-Raman spectrum:

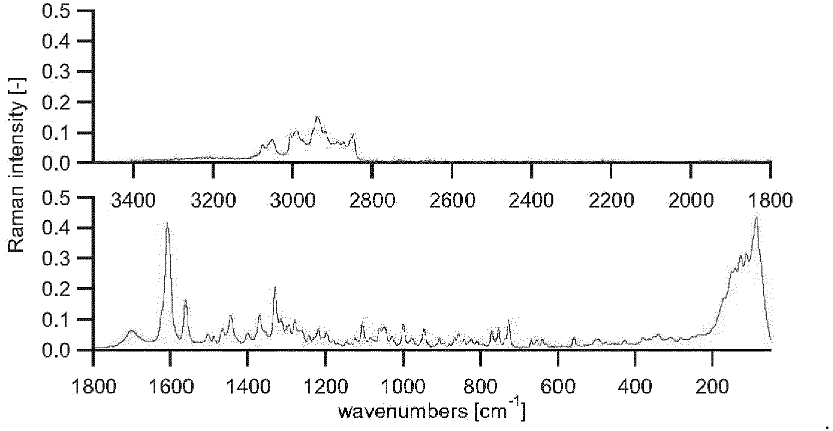

6. A hemifumarate salt of 1-(4-{1-[(E)-4-cyclohexyl-3-trifluoromethyl-benzyloxyimino]-ethyl}-2-ethyl-benzyl)-azetidine-3-carboxylic acid for use according to any of claims 1 to 3 wherein the salt comprises a Crystalline Form B, wherein the Crystalline Form B has the following FT-Raman spectrum:

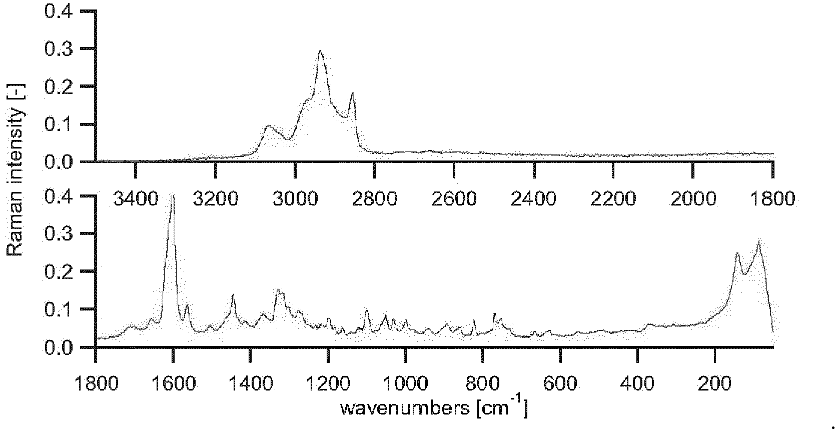

7. A hemifumarate salt of 1-(4-{1-[(E)-4-cyclohexyl-3-trifluoromethyl-benzyloxyimino]-ethyl}-2-ethyl-benzyl)-azetidine-3-carboxylic acid for use according to any of claims 1 to 3 wherein the salt comprises a Crystalline Form C, wherein the Crystalline Form C has the following FT-Raman spectrum:

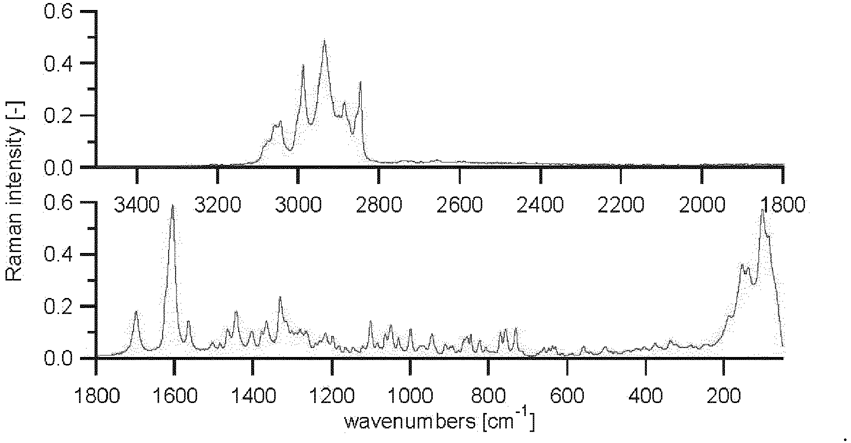

8. A hemifumarate salt of 1-(4-{1-[(E)-4-cyclohexyl-3-trifluoromethyl-benzyloxyimino]-ethyl}-2-ethyl-benzyl)-azetidine-3-carboxylic acid for use according to any of claims 1 to 3 wherein the salt comprises a Crystalline Form D, wherein the Crystalline Form D has the following FT-Raman spectrum:

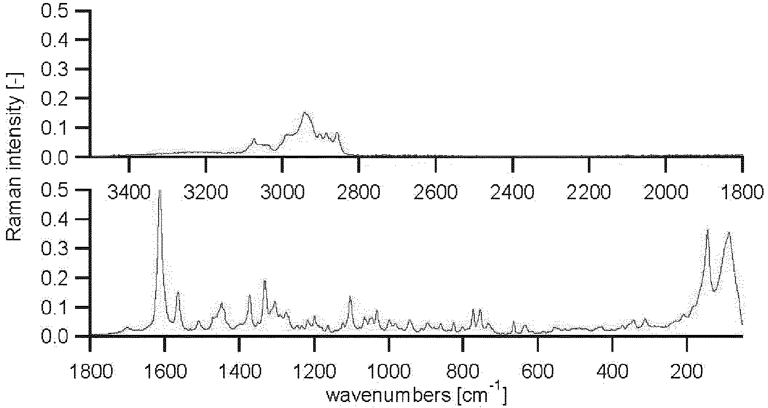

9. A hemifumarate salt of 1-(4-{1-[(E)-4-cyclohexyl-3-trifluoromethyl-benzyloxyimino]-ethyl}-2-ethyl-benzyl)-azetidine-3-carboxylic acid for use according to any of claims 1 to 3 wherein the salt comprises a Crystalline Form E, wherein the Crystalline Form E has the following FT-Raman spectrum:

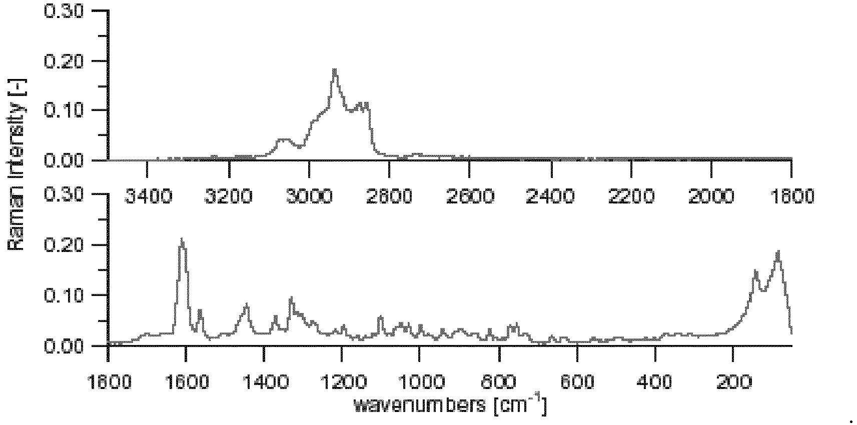

10. A pharmaceutical composition for use in treating or preventing diseases or disorders mediated by lymphocyte interactions according to claim 1, comprising a hemifumarate salt of 1-(4- {1-[(E)-4-cyclohexyl-3-trifluoromethyl-benzyloxyimino]-ethyl}-2-ethyl-benzyl)-azetidine-3-carboxylic acid in admixture with a pharmaceutically acceptable adjuvant, diluent or carrier.

11. A pharmaceutical combination for use in treating or preventing diseases or disorders mediated by lymphocyte interactions according to claim 1, comprising a hemifumarate salt of 1-(4- {1-[(E)-4-cyclohexyl-3-trifluoromethyl-benzyloxyimino]-ethyl}-2-ethyl-benzyl)-azetidine-3-carboxylic in combination with an immunosuppressant agent, an immunomodulating agent or other anti-inflammatory agents.

12. A pharmaceutical combination for use according to claim 11 in combinations with one or more of:
i. an aromatase inhibitor,
ii. an anti-estrogen, an anti-androgen (especially in the case of prostate cancer) or a gonadorelin agonist,
iii. a topoisomerase I inhibitor or a topoisomerase II inhibitor,
iv. a microtubule active agent, an alkylating agent, an antineoplastic antimetabolite or a platin compound,
v. a compound targeting/decreasing a protein or lipid kinase activity or a protein or lipid phosphatase activity, a further anti-angiogenic compound or a compound which induces cell differentiation processes,
vi. a bradykinin I receptor or an angiotensin II antagonist,
vii. a cyclooxygenase inhibitor, a bisphosphonate, a histone deacetylase inhibitor, a heparanase inhibitor, preferably PI-88, a biological response modifier, preferably a lymphokine or interferons, preferably interferon γ, an ubiquitination inhibitor, or an inhibitor which blocks anti-apoptotic pathways,
viii. an inhibitor of Ras oncogenic isoforms, preferably H-Ras, K-Ras or N-Ras, or a farnesyl transferase inhibitor, preferably L-744,832 or DK8G557,
ix. a telomerase inhibitor, preferably telomestatin,
x. a protease inhibitor, a matrix metalloproteinase inhibitor, a methionine aminopeptidase inhibitor, preferably bengamide or a derivative thereof, or a proteosome inhibitor, preferably PS-341, and/or
xi. a mTOR inhibitor.

13. A pharmaceutical combination for use according to claim 11 or 12 comprising one or more of:
a calcineurin inhibitor, preferably cyclosporin A or FK 506; a mTOR inhibitor, preferably rapamycin, 40-0-(2-hydroxyethyl)-rapamycin, CCI779, ABT578 or AP23573; an ascomycin having immunosuppressive properties, preferably ABT-281, ASM981; corticosteroids; cyclophosphamide; azathioprene; methotrexate; leflunomide; mizoribine; mycophenolic acid; mycophenolate mofetil; 15-deoxyspergualine or an immunosuppressive homologue, analogue or derivative thereof; immunosuppressive monoclonal antibodies, preferably monoclonal antibodies to leukocyte receptors, preferably MHC, CD2, CD3, CD4, CD7, CD8, CD25, CD28, CD40. CD45, CD58, CD80, CD86 or their ligands; other immunomodulatory compounds, preferably a recombinant binding molecule having at least a portion of the extracellular domain of CTLA4 or a mutant thereof, preferably an at least extracellular portion of CTLA4 or a mutant thereof joined to a non-CTLA4 protein sequence, preferably CTLA4Ig or a mutant thereof, preferably LEA29Y; adhesion molecule inhibitors, preferably LF A- I antagonists, ICAM-1 or -3 antagonists, VCAM-4 antagonists or VLA-4 antagonists; steroids, especially atamestane, exemestane and fonnestane and, in particular, non-steroids, especially amino glutethimide, roglethimide, pyridoglutethimide, trilostane, testolactone, ketokonazole, vorozole, fadrozole, anastrozole and letrozole; tamoxifen, fulvestrant, raloxifene and raloxifene hydrochloride; bicalutamide; abarelix, goserelin and goserelin acetate; topotecan, irinotecan, 9-nitrocamptothecin and the macromolecular camptothecin conjugate PNU-166148; the anthracyclines such as doxorubicin, daunorubicin, epirubicin, idarubicin and nemorubicin, the anthraquinones mitoxantrone and losoxantrone, and the podophillotoxines etoposide and teniposide; taxanes, preferably paclitaxel and docetaxel, vinca alkaloids, preferably vinblastine, especially vinblastine sulfate, vincristine especially vincristine sulfate, and vinorelbine, discodennolides and epothilones and derivatives thereof, preferably epothilone B or a derivative thereof; busulfan, chlorambucil, cyclophosphamide, ifosfamide, melphalan or nitrosourea; 5-fluorouracil, capecitabine, gemcitabine, cytarabine, fludarabine, thioguanine, methotrexate and edatrexate; carboplatin, cis-platin and oxaliplatin; protein tyrosine kinase and/or serine and/or threonine kinase inhibitors or lipid kinase inhibitors, preferably compounds targeting, decreasing or inhibiting the activity of the epidermal growth factor family of receptor tyrosine kinases (EGFR, ErbB2, ErbB3, ErbB4 as homo- or heterodimers), the vascular endothelial growth factor family of receptor tyrosine kinases (VEGFR), the platelet-derived growth factor-receptors (PDGFR), the fibroblast growth factor-receptors (FGFR), the insulin-like growth factor receptor 1 (IGF-IR), the Trk receptor tyrosine kinase family, the Axl receptor tyrosine kinase family, the Ret receptor tyrosine kinase, the KitlSCFR receptor tyrosine kinase, members of the c-Abl family and their genefusion products, preferably BCR-Abl, members of the protein kinase C (PKC) and Raf family of serine/threonine kinases, members of the MEK, SRC, JAK, FAK, PDK or PI(3) kinase family, or of the PI(3)-kinase-related kinase family, and/or members of the cyclin-dependent kinase family (CDK) and anti-angiogenic compounds having another mechanism for their activity, preferably unrelated to protein or lipid kinase inhibition; 1-(4-chloroanilino)-4-(4-pyridylmethyl)phthalazine or a pharmaceutically acceptable salt thereof, a N-aryl(thio) anthranilic acid amide derivative, preferably 2-[(4-pyridyl)methyl]amino-N-[3-methoxy-5-(trifluoromethyl)phenyl] benzamide or 2-[(1-oxido-4-pyridyl)methyl]amino-N-[3-trifluoromethylphenyl] benzamide, Angiostatin; Endostatin; anthranilic acid amides; ZD4190; ZD6474; SU5416; SU6668; or anti-VEGF antibodies or anti-VEGF receptor antibodies, preferably RhuMab; intact monoclonal antibodies, polyclonal antibodies, multispecific antibodies formed from at least 2 intact antibodies, and antibody fragments so long as they exhibit the desired biological activity; compounds, proteins or antibodies which inhibit members of the EGF receptor tyrosine kinase family, preferably EGF receptor, ErbB2, ErbB3 and ErbB4 or bind to EGF or EGF related ligands, or which have a dual inhibiting effect on the ErbB and VEGF receptor kinase and are preferably trastuzumab, cetuximab, Iressa, OSI-774, CI-1033, EKB-569, GW-2016, E1.1, E2.4, E2.5, E6.2, E6.4, E2.11, E6.3 or E7.6.3; a N-phenyl-2-pyrimidine-amine derivative, preferably imatinib; a N-phenyl-2-pyrimidine-amine derivative, preferably imatinib; PD180970; AG957; or NSC 680410; staurosporine derivatives, preferably midostaurin; UCN-Ol, safingol, BAY 43-9006, Bryostatin 1, Perifosine; Ilmofosine; RO 318220 and RO 320432; GO 6976; Isis 3521; or LY333531/LY379196; thalidomide and TNP- 470; inhibitors of phosphatase 1, phosphatase 2A, PTEN or CDC25, preferably okadaic acid or a derivative thereof; retinoic acid, α-, γ- or δ-tocopherol or α-, γ- or δ-tocotrienol; celecoxib, rofecoxib, etoricoxib, valdecoxib or a 5-alkyl-2-arylaminophenylacetic acid, preferably 5-methyl-2-(2'-chloro-6'-fluoroanilino)phenyl acetic acid; MS-275, SAHA, pyroxamide, FR-901228 or valproic acid; etridonic, clodronic, tiludronic, pamidronic, alendronic, ibandronic, risedronic and zoledronic acid; collagen peptidomimetic and non-petidomimetic inhibitors, tetracycline derivatives, preferably hydroxamate peptidomimetic inhibitor batimastat and its orally bioavailable analogue marimastat, prinomastat, BMS-279251, BAY 12-9566, TAA211 or AAJ996; rapamycin (sirolimus) or a derivative thereof, preferably 32-deoxorapamycin, 16-pent-2-ynyloxy-32-deoxorapamycin, 16-pent-2-ynyloxy-32(S)-dihydro-rapamycin, 16-pent-2-ynyloxy-32(S)-dihydro-40-0-(2-hydroxyethyl)-rapamycin and, more preferably, 40-0-(2-hydroxyethyl)-rapamycin; CCI779 or 40-[3-hydroxy-2-(hydroxymethyl)-2-methylpropanoate]-rapamycin or a pharmaceutically acceptable salt thereof, ABT578 or 40-(tetrazolyl)-rapamycin, particularly 40-epi-(tetrazolyl)-rapamycin, or rapalogs.

14. A pharmaceutical combination for use according to any of claims 11 to 13, wherein the combination is a fixed combination being administered as a single dosage.

## Patentansprüche

1. Hemifumaratsalz von 1-(4-{1-[(E)-4-Cyclohexyl-3-trifluormethyl-benzyloxyimino]ethyl}-2-ethylbenzyl)azetidin-3-carbonsäure zur Verwendung in der Behandlung oder Vorbeugung von durch Lymphozyteninteraktionen vermittelten Krankheiten oder Leiden, wobei die Krankheit oder das Leiden aus den Folgenden ausgewählt ist: akuter oder chronischer Abstoßung von Zellen-, Gewebe-Organallo- oder Xenotransplantaten oder verzögerter Transplantatfunktion, Graft-versus-Host-Krankheit, rheumatoider Arthritis, systemischem Lupus erythematodes, Hashimoto-Thyroidis, Myasthenia gravis, Diabetes-Typ I oder II und den damit verbundenen Störungen, Vasculitis, perniziöser Anämie, Sjoegren-Syndrom, Uveitis, Psoriasis, Graves-Ophthalmopathie, Alopecia areata; allergischen Erkrankungen, vorzugsweise allergischem Asthma, atopischer Dermatitis, allergischer Rhinitis/Konjunktivitis, allergischer Kontaktdermatitis; entzündlichen Erkrankungen, gegebenenfalls mit zugrundeliegenden Fehlreaktionen, vorzugsweise entzündlicher Darmerkrankung, Morbus Chrohn oder ulzerierender Kolitis, intrinsischem Asthma, entzündlicher Lungenschädigung, entzündlicher Leberschädigung, entzündlicher Glomerulumschädigung, Atherosklerose, Osteoarthritis, irritativer Kontaktdermatitis und weiteren Ekzemdermatiden, seborrhoischer Dermatitis, kutanen Manifestationen immuninduzierter Störungen, entzündlichen Augenerkrankungen, Keratokonjunktivitis, entzündlicher Myopathie; Myokarditis oder Hepatitis; Ischämie/Reperfusionsschädigung, vorzugsweise Herzinfarkt, Schlaganfall, Darmischämie, Nierenversagen oder hämorrhigischem Schock, traumatischem Schock; T-Zell-Lymphomen oder T-Zell-Leukämien; Infektionserkrankungen, vorzugsweise toxischem Schock, septischem Schock, Atemnotsyndrom beim Erwachsenen oder Virusinfektionen, vorzugsweise AIDS, Virus-Hepatitis, chronischer Bakterieninfektion; Muskelerkrankungen, vorzugsweise Polymyositis; oder seniler Demenz; Krebs, vorzugsweise Brustkrebs, oder als antiangiogenes Mittel; peripheren Neuropathien, insbesondere akuten oder chronischen demyelinisierenden Neuropathien wie Guillain-Barré-Syndrom (GBS), chronisch-entzündlicher demyelinisierender Polyradiculoneuropathie (CIDP), multifokaler Motorneuropathie mit Leitungsblockade (MMN), sowie paraproteinämischer demyelinisierender peripherer Neuropathie (PDN).

2. Hemifumaratsalz von 1-(4-{1-[(E)-4-Cyclohexyl-3-trifluormethyl-benzyloxyimino]ethyl}-2-ethylbenzyl)azetidin-3-carbonsäure zur Verwendung in der Behandlung oder Vorbeugung von durch Lymphozyteninteraktionen vermittelten Krankheiten oder Leiden, nach Anspruch 1, wobei es sich bei der Krankheit oder bei dem Leiden um Polymyositis handelt.

3. Hemifumaratsalz von 1-(4-{1-[(E)-4-Cyclohexyl-3-trifluormethyl-benzyloxyimino]ethyl}-2-ethylbenzyl)azetidin-3-carbonsäure zur Verwendung nach Anspruch 1 oder 2, wobei das Salz einen Kristallinitätsgrad von mehr als ungefähr 20%, vorzugsweise mehr als ungefähr 90%, aufweist.

4. Hemifumaratsalz von 1-(4-{1-[(E)-4-Cyclohexyl-3-trifluormethyl-benzyloxyimino]ethyl}-2-ethylbenzyl)azetidin-3-carbonsäure zur Verwendung nach einem der Ansprüche 1 bis 3, wobei das Salz eine Kristallform A umfasst, wobei die Kristallform A ein Röntgenpulverdiffraktionsmuster mit mindestens einem spezifischen Peak bei ungefähr 2-theta = 6,9°, 10,1°, 10,6°, 12,1°, 17,5°, 18,1° oder 20,7° aufweist.

5. Hemifumaratsalz von 1-(4-{1-[(E)-4-Cyclohexyl-3-trifluormethyl-benzyloxyimino]ethyl}-2-ethylbenzyl)azetidin-3-carbonsäure zur Verwendung nach einem der Ansprüche 1 bis 4, wobei das Salz eine Kristallform A umfasst, wobei die Kristallform A das folgende FT-Raman-Spektrum aufweist:

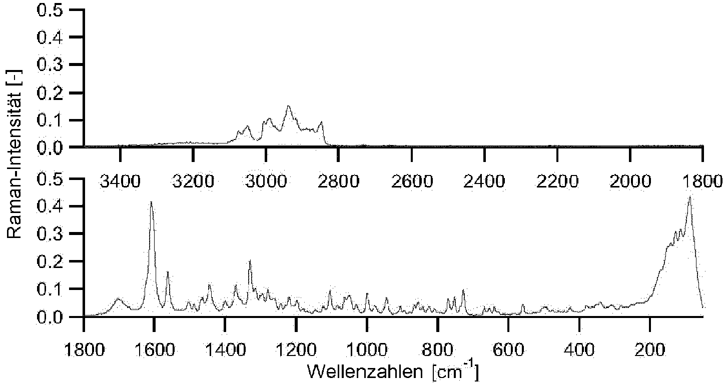

6. Hemifumaratsalz von 1-(4-{1-[(E)-4-Cyclohexyl-3-trifluormethyl-benzyloxyimino]ethyl}-2-ethylbenzyl)azetidin-3-carbonsäure zur Verwendung nach einem der Ansprüche 1 bis 3, wobei das Salz eine Kristallform B umfasst, wobei die Kristallform B das folgende FT-Raman-Spektrum aufweist:

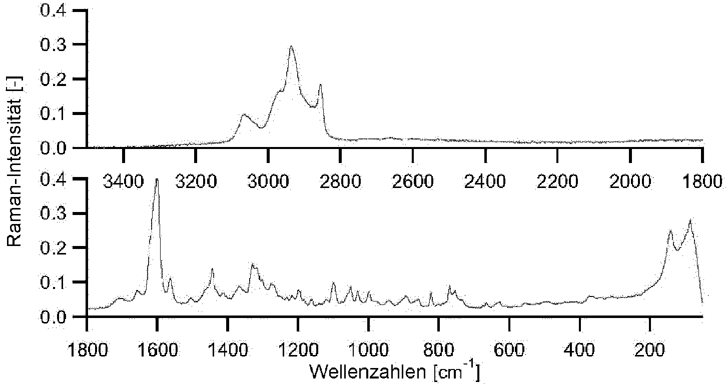

7. Hemifumaratsalz von 1-(4-{1-[(E)-4-Cyclohexyl-3-trifluormethyl-benzyloxyimino]ethyl}-2-ethylbenzyl)azetidin-3-carbonsäure zur Verwendung nach einem der Ansprüche 1 bis 3, wobei das Salz eine Kristallform C umfasst, wobei die Kristallform C das folgende FT-Raman-Spektrum aufweist:

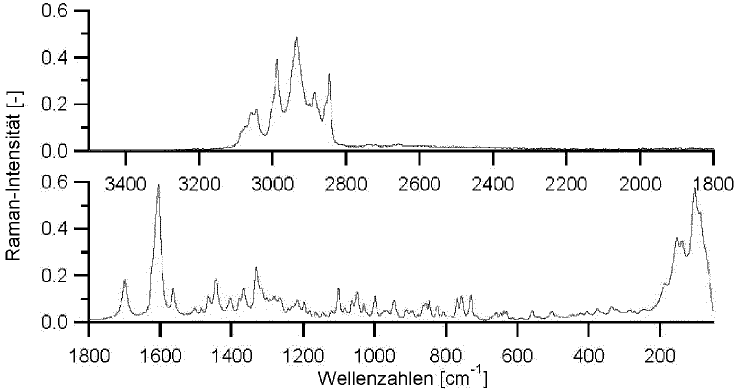

8. Hemifumaratsalz von 1-(4-{1-[(E)-4-Cyclohexyl-3-trifluormethyl-benzyloxyimino]ethyl}-2-ethylbenzyl)azetidin-3-carbonsäure zur Verwendung nach einem der Ansprüche 1 bis 3, wobei das Salz eine Kristallform D umfasst, wobei die Kristallform D das folgende FT-Raman-Spektrum aufweist:

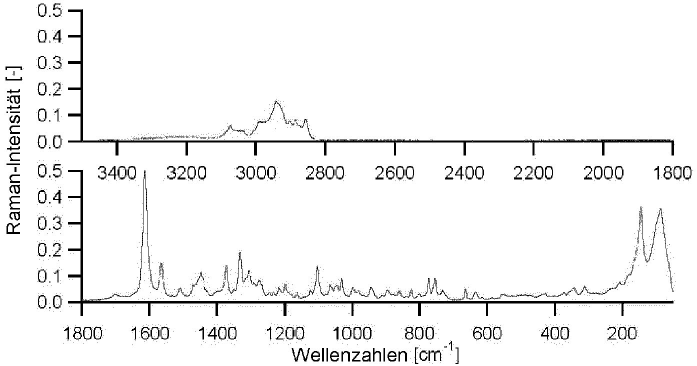

9. Hemifumaratsalz von 1-(4-{1-[(E)-4-Cyclohexyl-3-trifluormethyl-benzyloxyimino]ethyl}-2-ethylbenzyl)azetidin-3-carbonsäure zur Verwendung nach einem der Ansprüche 1 bis 3, wobei das Salz eine Kristallform E umfasst, wobei die Kristallform E das folgende FT-Raman-Spektrum aufweist:

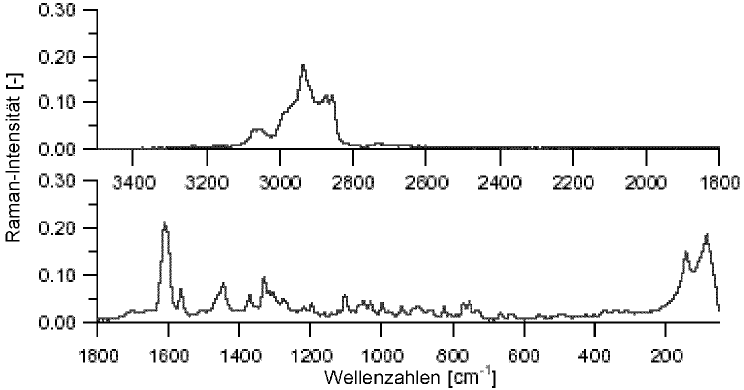

10. Pharmazeutische Zusammensetzung zur Verwendung in der Behandlung oder Vorbeugung von durch Lymphozyteninteraktionen vermittelten Krankheiten oder Leiden nach Anspruch 1, umfassend ein Hemifumaratsalz von 1-(4-{1-[(E)-4-Cyclohexyl-3-trifluormethyl-benzyloxyimino]ethyl}-2-ethylbenzyl)azetidin-3-carbonsäure in Abmischung mit einem pharmazeutisch annehmbaren Hilfsstoff, Verdünnungsmittel oder Träger.

11. Pharmazeutische Kombination zur Verwendung in der Behandlung oder Vorbeugung von durch Lymphozyteninteraktionen vermittelten Krankheiten oder Leiden nach Anspruch 1, umfassend ein Hemifumaratsalz von 1-(4-{1-[(E)-4-Cyclohexyl-3-trifluormethyl-benzyloxyimino]ethyl}-2-ethylbenzyl)azetidin-3-carbon in Kombination mit einem immunsuppremierenden Mittel, einem immunmodulierenden Mittel oder anderen entzündungshemmenden Mitteln.

12. Pharmazeutische Kombination zur Verwendung nach Anspruch 11 in Kombinationen mit einem/einer oder mehreren der Folgenden:
i. einem Aromatase-Hemmer,
ii. einem Antiöstrogen, einem Antiandrogen (insbesondere bei Prostatakrebs) oder einem Gonadorelin-Agonisten,
iii. einem Topoisomerase-I-Hemmer oder einem Topoisomerase-II-Hemmer,
iv. einem Mikrotubuli-Wirkstoff, einem Alkylierungsmittel, einem antineoplastischen Antimetaboliten oder einer Platinverbindung,
v. einer Verbindung, der ein Protein oder eine Lipidkinaseaktivität oder ein Protein oder eine Lipidphosphataseaktivität als Angriffspunkt dient bzw. die diese vermindert, einer weiteren antiangiogenen Verbindung oder einer Verbindung, die Zelldifferenzierungsvorgänge induziert,
vi. einem Bradykinin-I-Rezeptor oder einem Angiotensin-II-Antagonisten,
vii. einem Cyclooxygenase-Hemmer, einem Bisphosphonat, einem Histondeacetylase-Hemmer, einem Heparanase-Hemmer, vorzugsweise PI-88, einem Modifikator der biologischen Reaktion, vorzugsweise einem Lymphokin oder Interferonen, vorzugsweise Interferon γ, einem Ubiquitinierungs-Hemmer oder einem Hemmer, der antiapoptotische Wege blockiert,
viii. einem Hemmer von onkogenen Ras-Isoformen, vorzugsweise H-Ras, K-Ras oder N-Ras, oder einem Farnesyltransferase-Hemmer, vorzugsweise L-744,832 oder DK8G557,
ix. einem Telomerase-Hemmer, vorzugsweise Telomestatin,
x. einem Protease-Hemmer, einem Matrix-Metalloproteinase-Hemmer, einem Methioninaminopeptidase-Hemmer, vorzugsweise Bengamid oder einem Derivativ davon, oder einem Proteosomenhemmer, vorzugsweise PS-341, und/oder
xi. einem mTOR-Hemmer.

13. Pharmazeutische Kombination zur Verwendung nach Anspruch 11 oder 12, die eines/einen/eine oder mehrere der Folgenden umfasst:
einen Calcineurin-Hemmer, vorzugsweise Cyclosporin A oder FK 506; einen mTOR-Hemmer, vorzugsweise Rapamycin, 40-0-(2-Hydroxyethyl)rapamycin, CCI779, ABT578 oder AP23573; ein Ascomycin mit immunsuppremierenden Eigenschaften, vorzugsweise ABT-281, ASM981; Corticosteroide; Cyclophosphamid; Azathiopren; Methotrexat; Leflunomid; Mizoribin; Mycophenolsäure; Mycophenolat Mofetil; 15-Desoxyspergualin oder ein immunsuppremierendes Homolog, Analog oder Derivativ davon; immunsuppremierende monoklonale Antikörper, vorzugsweise monoklonale Antikörper gegen Leukozytenrezeptoren, vorzugsweise MHC, CD2, CD3, CD4, CD7, CD8, CD25, CD28, CD40, CD45, CD58, CD80, CD86 oder ihre Liganden; andere immunmodulierende Verbindungen, vorzugsweise ein rekombinante Bindungsmolekül mit mindestens einem Teil der extrazellulären Domäne von CTLA4 oder eine Mutante davon, vorzugsweise mindestens einen extrazellulären Teil von CTLA4 oder eine Mutante davon in Verknüpfung mit einer Nicht-CTLA4-Proteinsequenz, vorzugsweise CTLA4Ig oder eine Mutante davon, vorzugsweise LEA29Y; Adhäsionsmolekülhemmer, vorzugsweise LF A-I-Antagonisten, ICAM-1- oder -3-Antagonisten, VCAM-4-Antagonisten oder VLA-4-Antagonisten; Steroide, speziell Atamestan, Exemestan und Fonnestan und insbesondere Nicht-Steroide, speziell Aminoglutethimid, Roglethimid, Pyridoglutethimid, Trilostan, Testolacton, Ketokonazol, Vorozol, Fadrozol, Anastrozol und Letrozol; Tamoxifen, Fulvestrant, Raloxifen und Raloxifen-Hydrochlorid; Bicalutamid; Abarelix, Goserelin und Goserelin-Acetat; Topotecan, Irinotecan, 9-Nitrocamptothecin und das makromolekulare Camptothecinkonjugat PNU-166148; die Anthracycline wie Doxorubicin, Daunorubicin, Epirubicin, Idarubicin und Nemorubicin, die Anthraquinone Mitoxantron und Losoxantron, und die Podophillotoxine Etoposid und Teniposid; Taxane, vorzugsweise Paclitaxel und Docetaxel, Vinca-Alkaloide, vorzugsweise Vinblastin, speziell Vinblastinsulfat, Vincristin, speziell Vincristinsulfat, und Vinorelbin, Discodennolide und Epothilone und Derivative davon, vorzugsweise Epothilon B oder ein Derivativ davon; Busulfan, Chlorambucil, Cyclophosphamid, Ifosfamid, Melphalan oder Nitrosoharnstoff; 5-Fluoruracil, Capecitabin, Gemcitabin, Cytarabin, Fludarabin, Thioguanin, Methotrexat und Edatrexat; Carboplatin, Cisplatin und Oxaliplatin; Proteintyrosinkinase- und/oder- Serin- und/oder- Threoninkinasehemmer oder Lipidkinasehemmer, vorzugsweise Verbindungen, denen die Aktivität der Epidermiswachstumsfaktorfamilie der Rezeptortyrosinkinasen (EGFR, ErbB2, ErbB3, ErbB4 als Homo- oder Heterodimere) als Angriffspunkt dient bzw. die diese vermindern oder hemmen, die Gefäßendothelwachstumsfaktorfamilie der Rezeptortyrosinkinasen (VEGFR), die Rezeptoren des Platelet-Derived Growth Factor (PDGFR), die Rezeptoren des Fibroblast Growth Factor (FGFR), die Rezeptor 1 des Insulin-Like Growth Factor (IGF-IR), den Trk-Rezeptor-Tyrosinkinasefamilie, die Axl-Rezeptor-Tyrosinkinasefamilie, die Ret- Rezeptor-Tyrosinkinase, die KitlSCFR-Rezeptor-Tyrosinkinase, Mitglieder der c-Abl-Familie und ihre Genfusionsprodukte, vorzugsweise BCR-Abl, Mitgliedern der Proteinkinase C (PKC) und der Raf-Familie der Serin/Threoninkinasen, Mitglieder der MEK-, SRC-, JAK-, FAK-, PDK- oder PI(3)-Kinasefamilie, oder der PI(3)-Kinase-Related Kinase-Familie, und/oder Mitglieder der Cyclin-Dependent Kinase-Familie (CDK) und anti-angiogene Verbindungen mit einem anderen Mechanismus für ihre Wirkung, die vorzugsweise mit der Protein- oder Lipidkinasehemmung nicht verwandt ist; 1-(4-Chloranilino)-4-(4-pyridylmethyl)phthalazin oder ein pharmazeutisch annehmbares Salz davon, ein N-Aryl(thio)anthranilsäureamidderivativ, vorzugsweise 2-[(4-Pyridyl)methyl]amino-N-[3-methoxy-5-(trifluormethyl)phenyl]benzamid oder 2-[(1-Oxido-4-pyridyl)methyl]amino-N-[3-trifluormethylphenyl] benzamid, Angiostatin; Endostatin; Anthranilsäureamide; ZD4190; ZD6474; SU5416; SU6668; oder anti-VEGF-Antikörper oder anti-VEGF-Rezeptorantikörper, vorzugsweise RhuMab; intakte monoklonale Antikörper, polyklonale Antikörper, multispezifische Antikörper, die aus mindestens 2 intakten Antikörpern gebildet werden, und Antikörperfragmente, die lang genug sind, dass sie die gewünschte biologische Wirkung aufweisen; Verbindungen, Proteine oder Antikörper, die Mitglieder der EGF-Rezeptor-Tyrosinkinasefamilie, vorzugsweise EGF-Rezeptor, ErbB2, ErbB3 und ErbB4, hemmen oder an EGF oder mit EGF verwandte Liganden binden, oder die eine Doppelhemmwirkung auf die ErbB- und VEGF-Rezeptorkinase ausüben und vorzugsweise Trastuzumab, Cetuximab, Iressa, OSI-774, CI-1033, EKB-569, GW-2016, E1.1, E2.4, E2.5, E6.2, E6.4, E2.11, E6.3 oder E7.6.3 sind; ein N-Phenyl-2-pyrimidinaminderivativ, vorzugsweise Imatinib; ein N-Phenyl-2-pyrimidinamin-derivativ, vorzugsweise Imatinib; PD180970; AG957; oder NSC 680410; Staurosporinderivative, vorzugsweise Midostaurin; UCN-Ol, Safingol, BAY 43-9006, Bryostatin 1, Perifosin; Ilmofosin; RO 318220 und RO 320432; GO 6976; Isis 3521; oder LY333531/LY379196; Thalidomid und TNP-470; Hemmer der Phosphatase 1, Phosphatase 2A, PTEN oder CDC25, vorzugsweise Okadasäure oder ein Derivat davon; Retinsäure, α-, γ- oder δ-Tocopherol oder α-, γ- oder δ-Tocotrienol; Celecoxib, Rofecoxib, Etoricoxib, Valdecoxib oder eine 5-Alkyl-2-arylaminophenylessigsäure, vorzugsweise 5-Methyl-2-(2'-chlor-6'-fluoranilino)phenylessigsäure; MS-275, SAHA, Pyroxamid, FR-901228 oder Valproinsäure; Etridonsäure, Clodronsäure, Tiludronsäure, Pamidronsäure, Alendronsäure, Ibandronsäure, Risedronsäure und Zoledronsäure; Hemmer von Collagenpeptidomimetika und Nicht-Petidomimetika; Tetracyclinderivative, vorzugsweise den Hydroxamat-Peptidomimetikum-Hemmer Batimastat und sein oral biologisch verfügbares Analog Marimastat, Prinomastat, BMS-279251, BAY 12-9566, TAA211 oder AAJ996; Rapamycin (sirolimus) oder ein Derivativ davon, vorzugsweise 32-Desoxorapamycin, 16-Pent-2-inyloxy-32-desoxorapamycin, 16-Pent-2-inyloxy-32(S)-dihydrorapamycin, 16-Pent-2-inyloxy-32(S)-dihydro-40-0-(2-hydroxyethyl)rapamycin und, stärker bevorzugt, 40-0-(2-Hydroxyethyl)rapamycin; CCI779 oder 40-[3-Hydroxy-2-(hydroxymethyl)-2-methylpropanoat]rapamycin oder ein pharmazeutisch annehmbares Salz davon, ABT578 oder 40-(Tetrazolyl)-rapamycin, insbesondere 40-epi-(Tetrazolyl)rapamycin, oder Rapalogen.

14. Pharmazeutische Kombination zur Verwendung nach einem der Ansprüche 11 bis 13, wobei es sich bei der Kombination um eine fixe Kombination, die als Einzeldosis verabreicht wird, handelt.

## Revendications

1. Sel d'hémifumarate d'acide 1-(4-{1-[(E)-4-cyclohexyl-3-trifluorométhyl-benzyloxyimino]-éthyl}-2-éthyl-benzyl)-azétidine-3-carboxylique pour utilisation dans le traitement ou la prévention de maladies ou troubles médié(e)s par des interactions des lymphocytes, la maladie ou le trouble étant choisi(e) parmi un rejet aigu ou chronique d'allo- ou xénogreffes de cellules, de tissu ou d'organe ou une fonction de greffe retardée, la maladie du greffon contre l'hôte ; la polyarthrite rhumatoïde, le lupus érythémateux disséminé, la thyroïdis de Hashimoto, la myasthénie grave, le diabète de type I ou II et les troubles associés à celui-ci, la vascularite, l'anémie pernicieuse, le syndrome de Sjoegren, l'uvéite, le psoriasis, l'ophtalmopathie de Graves, la pelade ; des maladies allergiques, de préférence l'asthme allergique, la dermatite atopique, la rhinite/conjonctivite allergique, la dermatite allergique de contact ; des maladies inflammatoires, éventuellement avec des réactions aberrantes sous-jacentes, de préférence une maladie intestinale inflammatoire, la maladie de Crohn ou la rectocolite hémorragique, l'asthme intrinsèque, une lésion inflammatoire pulmonaire, une lésion inflammatoire hépatique, une lésion glomérulaire inflammatoire, l'athérosclérose, l'arthrose, la dermatite de contact irritante et d'autres dermatites eczémateuses, la dermatite séborrhéique, les manifestations cutanées de troubles à médiation immunologique, une maladie inflammatoire oculaire, la kératoconjonctivite, une myopathie inflammatoire; une myocardite ou une hépatite; une lésion d'ischémie / reperfusion, de préférence un infarctus du myocarde, un accident vasculaire cérébral, une ischémie intestinale, une insuffisance rénale ou un choc hémorragique, un choc traumatique ; des lymphomes T ou des leucémies à cellules T ; des maladies infectieuses, de préférence un choc toxique, un choc septique, un syndrome de détresse respiratoire de l'adulte ou des infections virales, de préférence le SIDA, l'hépatite virale, une infection bactérienne chronique ; des maladies musculaires, de préférence la polymyosite ; ou la démence sénile; un cancer, de préférence un cancer du sein, ou en tant qu'agent antiangiogénique ; des neuropathies périphériques, en particulier des neuropathies démyélinisantes aiguës ou chroniques telles que le syndrome de Guillain-Barré (GBS), la polyradiculoneuropathie démyélinisante inflammatoire chronique (PIDC), la neuropathie motrice multifocale avec bloc de conduction (NMM) et la neuropathie périphérique démyélinisante paraprotéinémique (NPD).

2. Sel d'hémifumarate d'acide 1-(4-{1-[(E)-4-cyclohexyl-3-trifluorométhyl-benzyloxyimino]-éthyl}-2-éthyl-benzyl)-azétidine-3-carboxylique pour utilisation dans le traitement ou la prévention de maladies ou troubles médiés par des interactions des lymphocytes selon la revendication 1, la maladie ou le trouble étant la polymyosite.

3. Sel d'hémifumarate d'acide 1-(4-{1-[(E)-4-cyclohexyl-3-trifluorométhyl-benzyloxyimino]-éthyl}-2-éthyl-benzyl)-azétidine-3-carboxylique pour utilisation selon les revendications 1 à 2, ledit sel ayant un degré de cristallinité supérieur à environ 20 %, de préférence supérieur à environ 90 %.

4. Sel d'hémifumarate d'acide 1-(4-{1-[(E)-4-cyclohexyl-3-trifluorométhyl-benzyloxyimino]-éthyl}-2-éthyl-benzyl)-azétidine-3-carboxylique pour utilisation selon l'une quelconque des revendications 1 à 3, ledit sel comprenant une forme cristalline A, la forme cristalline A ayant un diagramme de diffraction des rayons X sur poudre avec au moins un pic spécifique à environ 2-thêta = 6,9°, 10,1°, 10,6°, 12,1°, 17,5°, 18,1° ou 20,7°.

5. Sel d'hémifumarate d'acide 1-(4-{1-[(E)-4-cyclohexyl-3-trifluorométhyl-benzyloxyimino]-éthyl}-2-éthyl-benzyl)-azétidine-3-carboxylique pour utilisation selon l'une quelconque des revendications 1 à 4, le sel comprenant une forme cristalline A, la forme cristalline A présentant le spectre FT-Raman suivant :

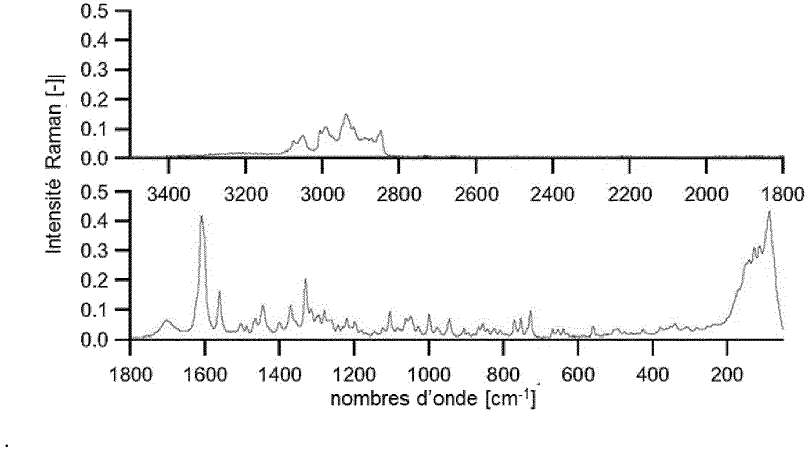

6. Sel d'hémifumarate d'acide 1-(4-{1-[(E)-4-cyclohexyl-3-trifluorométhyl-benzyloxyimino]-éthyl}-2-éthyl-benzyl)-azétidine-3-carboxylique pour utilisation selon l'une quelconque des revendications 1 à 3, le sel comprenant une forme cristalline B, la forme cristalline B présentant le spectre FT-Raman suivant :

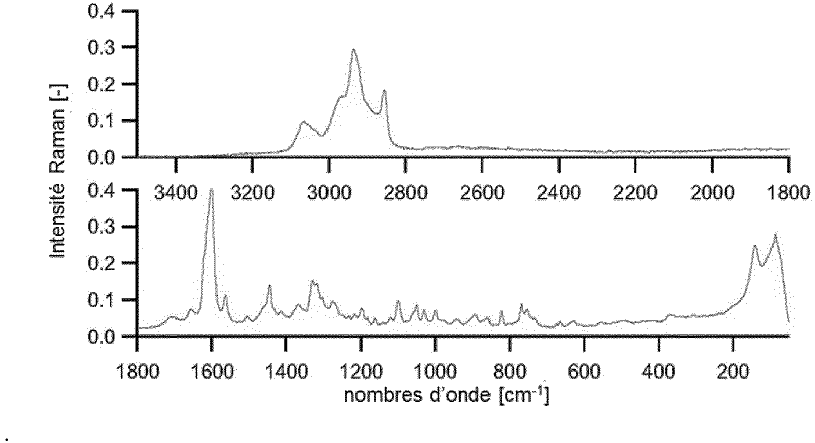

7. Sel d'hémifumarate d'acide 1-(4-{1-[(E)-4-cyclohexyl-3-trifluorométhyl-benzyloxyimino]-éthyl}-2-éthyl-benzyl)-azétidine-3-carboxylique pour utilisation selon l'une quelconque des revendications 1 à 3, le sel comprenant une forme cristalline C, la forme cristalline C présentant le spectre FT-Raman suivant :

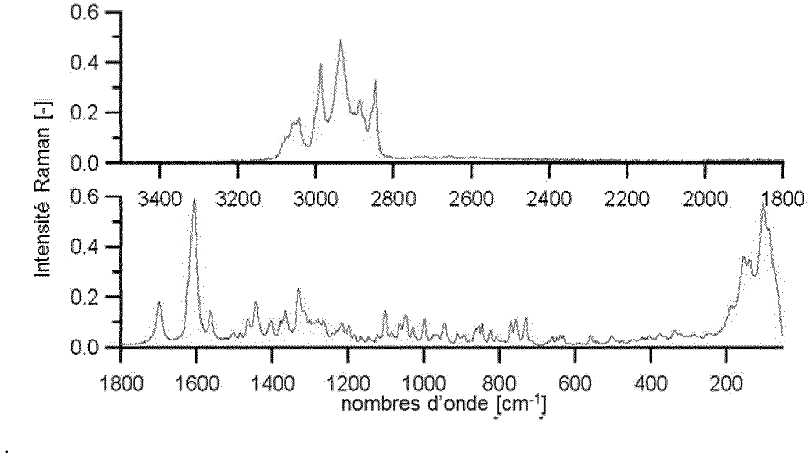

8. Sel d'hémifumarate d'acide 1-(4-{1-[(E)-4-cyclohexyl-3-trifluorométhyl-benzyloxyimino]-éthyl}-2-éthyl-benzyl)-azétidine-3-carboxylique pour utilisation selon l'une quelconque des revendications 1 à 3, le sel comprenant une forme cristalline D, la forme cristalline D présentant le spectre FT-Raman suivant :

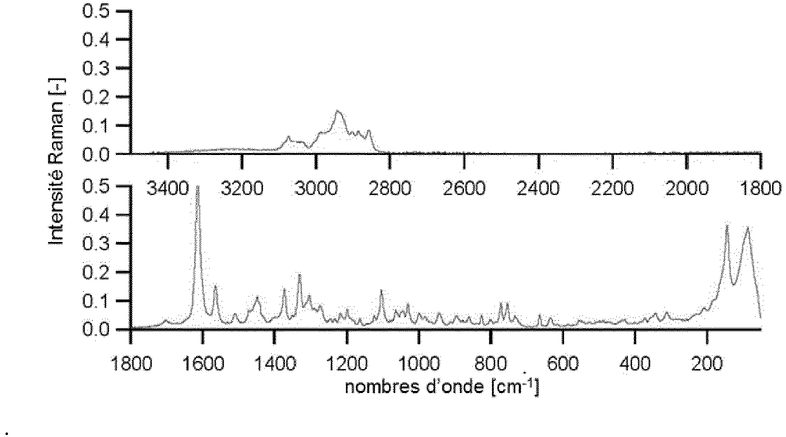

9. Sel d'hémifumarate d'acide 1-(4-{1-[(E)-4-cyclohexyl-3-trifluorométhyl-benzyloxyimino]-éthyl}-2-éthyl-benzyl)-azétidine-3-carboxylique pour utilisation selon l'une quelconque des revendications 1 à 3, le sel comprenant une forme cristalline E, la forme cristalline E présentant le spectre FT-Raman suivant :

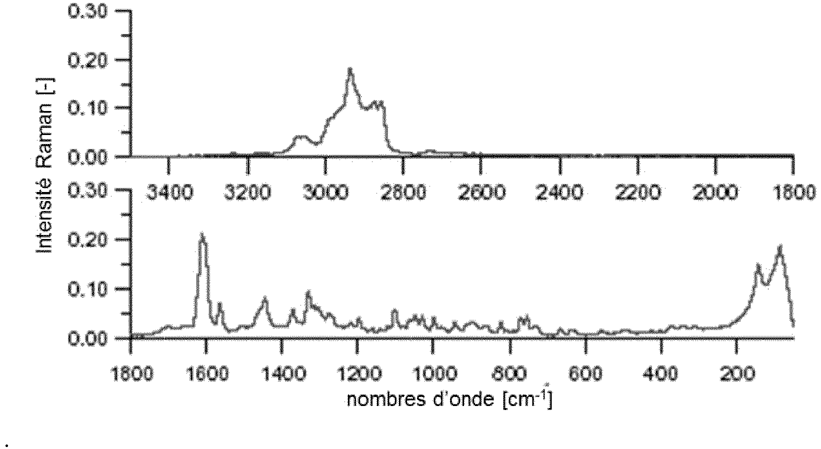

10. Composition pharmaceutique pour utilisation dans le traitement ou la prévention de maladies ou troubles médiés par des interactions des lymphocytes selon la revendication 1, comprenant un sel d'hémifumarate d'acide 1-(4-{1-[(E)-4-cyclohexyl-3-trifluorométhyl-benzyloxyimino]-éthyl}-2-éthyl-benzyl)-azétidine-3-carboxylique en mélange avec un adjuvant, diluant ou véhicule pharmaceutiquement acceptable.

11. Combinaison pharmaceutique pour utilisation dans le traitement ou la prévention de maladies ou troubles médiés par des interactions des lymphocytes selon la revendication 1, comprenant un sel d'hémifumarate de 1-(4-{1-[(E)-4-cyclohexyl-3-trifluorométhyl-benzyloxyimino]-éthyl}-2-éthyl-benzyl)-azétidine-3-carboxylique en combinaison avec un agent immunosuppresseur, un agent immunomodulateur ou d'autres agents anti-inflammatoires.

12. Combinaison pharmaceutique pour utilisation selon la revendication 11 dans des combinaisons avec l'un ou plusieurs parmi :
i. un inhibiteur d'aromatase,
ii. un anti-oestrogène, un antiandrogène (en particulier dans le cas du cancer de la prostate) ou un agoniste de gonadoréline,
iii. un inhibiteur de topoisomérase I ou un inhibiteur de topoisomérase II,
iv. un agent actif sur les microtubules, un agent d'alkylation, un antimétabolite antinéoplasique ou un composé de platine,
v. un composé ciblant/diminuant une activité protéine ou lipide kinase ou une activité protéine ou lipide phosphatase, un autre composé antiangiogénique ou un composé qui induit des processus de différenciation cellulaire,
vi. un récepteur de bradykinine I ou un antagoniste d'angiotensine II,
vii. un inhibiteur de cyclooxygénase, un bisphosphonate, un inhibiteur d'histone désacétylase, un inhibiteur d'héparanase, de préférence PI-88, un modificateur de réponse biologique, de préférence une lymphokine ou des interférons, de préférence l'interféron γ, un inhibiteur d'ubiquitination ou un inhibiteur qui bloque les voies anti-apoptose,
viii. un inhibiteur des isoformes oncogènes de Ras, de préférence H-Ras, K-Ras ou N-Ras ou un inhibiteur de farnésyl-transférase, de préférence L-744,832 ou DK8G557,
ix. un inhibiteur de télomérase, de préférence la télomestatine,
x. un inhibiteur de protéase, un inhibiteur de métalloprotéinase de matrice, un inhibiteur de méthionine aminopeptidase, de préférence le bengamide ou un dérivé de celui-ci, ou un inhibiteur de protéosome, de préférence PS-341 et/ou
xi. un inhibiteur de mTOR.

13. Combinaison pharmaceutique pour utilisation selon la revendication 11 ou 12 comprenant l'un ou plusieurs parmi :
un inhibiteur de calcineurine, de préférence la cyclosporine A ou FK 506 ; un inhibiteur de mTOR, de préférence la rapamycine, la 40-0-(2-hydroxyéthyl)-rapamycine, CCI779, ABT578 ou AP23573 ; une ascomycine ayant des propriétés immunosuppressives, de préférence ABT-281, ASM981 ; des corticostéroïdes ; le cyclophosphamide ; l'azathioprène ; le méthotrexate ; le léflunomide ; la mizoribine ; l'acide mycophénolique ; le mycophénolate mofétil ; la 15-désoxyspergualine ou un homologue immunosuppresseur, un analogue ou un dérivé de celui-ci ; des anticorps monoclonaux immunosuppresseurs, de préférence des anticorps monoclonaux contre des récepteurs leucocytaires, de préférence CMH, CD2, CD3, CD4, CD7, CD8, CD25, CD28, CD40, CD45, CD58, CD80, CD86 ou leurs ligands ; d'autres composés immunomodulateurs, de préférence une molécule de liaison recombinante comportant au moins une partie du domaine extracellulaire de CTLA4 ou d'un mutant de celle-ci, de préférence une partie au moins extracellulaire de CTLA4 ou un mutant de celle-ci assemblée à une séquence de protéine non-CTLA4, de préférence CTLA4Ig ou un mutant de celui-ci, de préférence LEA29Y ; des inhibiteurs de molécule d'adhésion, de préférence des antagonistes de LF A-I, des antagonistes de ICAM-1 ou -3, des antagonistes de VCAM-4 ou des antagonistes de VLA-4 ; des stéroïdes, en particulier l'atamestane, l'exémestane et le fonnestane et, en particulier, des non-stéroïdes, en particulier l'amino-glutéthimide, le rogléthimide, le pyridoglutéthimide, le trilostane, la testolactone, le kétokonazole, le vorozole, le fadrozole, l'anastrozole et le létrozole ; le tamoxifène, le fulvestrant, le raloxifène et le chlorhydrate de raloxifène ; le bicalutamide ; l'abarélix, la goséréline et l'acétate de goséréline ; le topotécan, l'irinotécan, la 9-nitrocamptothécine et le conjugué de camptothécine macromoléculaire PNU-166148 ; des anthracyclines telles que la doxorubicine, la daunorubicine, l'épirubicine, l'idarubicine et la néorubicine, les anthraquinones mitoxantrone et losoxantrone, et les podophillotoxines étoposide et téniposide ; des taxanes, de préférence le paclitaxel et le docétaxel, des alcaloïdes de pervenche, de préférence la vinblastine, en particulier le sulfate de vinblastine, la vincristine en particulier le sulfate de vincristine, et la vinorelbine, des discodennolides et des épothilones et leurs dérivés, de préférence l'épothilone B ou un dérivé de celle-ci ; le busulfan, le chlorambucil, le cyclophosphamide, l'ifosfamide, le melphalan ou la nitrosourée ; le 5-fluorouracile, la capécitabine, la gemcitabine, la cytarabine, la fludarabine, la thioguanine, le méthotrexate et l'édatrexate ; le carboplatine, le cisplatine et l'oxaliplatine ; des inhibiteurs de protéine tyrosine kinase et/ou de sérine et/ou thréonine kinase ou des inhibiteurs de lipide kinase, de préférence des composés ciblant, diminuant ou inhibant l'activité de la famille du facteur de croissance épidermique des récepteurs tyrosine kinases (EGFR, ErbB2, ErbB3 et ErbB4 sous forme d'homo- ou hétérodimères), la famille du facteur de croissance endothélial vasculaire des récepteurs tyrosine kinases (VEGFR), les récepteurs de facteur de croissance dérivés des plaquettes (PDGFR), les récepteurs de facteur de croissance des fibroblastes (FGFR), le récepteur de facteur de croissance insulinomimétique 1 (IGF-IR), la famille du récepteur tyrosine kinase Trk, la famille du récepteur tyrosine kinase Axl, le récepteur tyrosine kinase Ret, le récepteur tyrosine kinase KitlSCFR, des membres de la famille c-Abl et leurs produits de fusion de gène, de préférence BCR-Abl, des membres de la famille de protéine kinase C (PKC) et Raf de sérine/thréonine kinases, des membres de la famille de kinases MEK, SRC, JAK, FAK, PDK ou PI (3), ou de la famille de kinases apparentées à la kinase PI(3) et/ou des membres de la famille de kinases cycline-dépendantes (CDK) et des composés antiangiogéniques ayant un autre mécanisme pour leur activité, de préférence sans rapport avec l'inhibition de protéine ou lipide kinase ; la 1-(4-chloroanilino)-4-(4-pyridylméthyl)phtalazine ou un sel pharmaceutiquement acceptable de celle-ci, un dérivé d'amide d'acide N-aryl(thio)anthranilique, de préférence, le 2-[(4-pyridyl)méthyl]amino-N-[3-méthoxy-5-(trifluorométhyl)phényl]benzamide ou le 2-[(1-oxido-4-pyridyl)méthyl]amino-N-[3-trifluorométhylphenyl] benzamide, l'angiostatine ; l'endostatine ; des amides d'acide anthranilique ; ZD4190 ; ZD6474 ; SU5416 ; SU6668 ; ou des anticorps anti-VEGF ou des anticorps anti-récepteur de VEGF, de préférence RhuMab ; des anticorps monoclonaux intacts, des anticorps polyclonaux, des anticorps multispécifiques formés d'au moins 2 anticorps intacts, et des fragments d'anticorps dans la mesure où ils présentent l'activité biologique souhaitée ; des composés, des protéines ou des anticorps qui inhibent des membres de la famille du récepteur tyrosine kinase EGF, de préférence un récepteur EGF, ErbB2, ErbB3 et ErbB4 ou se lient à EGF ou des ligands apparentés à EGF, ou qui ont un double effet inhibiteur sur le récepteur kinase ErbB et VEGF et sont de préférence le trastuzumab, le cétuximab, Iressa, OSI-774, CI-1033, EKB-569, GW-2016, E1.1 E2.4, E2.5, E6.2, E6.4, E2.11, E6.3 ou E7.6.3 ; un dérivé de N-phényl-2-pyrimidine-amine, de préférence l'imatinib ; un dérivé de N-phényl-2-pyrimidine-amine, de préférence l'imatinib ; PD180970 ; AG957 ; ou NSC 680410 ; des dérivés de staurosporine, de préférence la midostaurine ; UCN-Ol, le safingol, BAY 43-9006, la bryostatine 1, la périfosine ; la Ilmofosine ; RO 318220 et RO 320432 ; GO 6976 ; Isis 3521 ; ou LY333531 / LY379196 ; le thalidomide et TNP-470 ; les inhibiteurs de phosphatase 1, phosphatase 2A, PTEN ou CDC25, de préférence l'acide okadaïque ou un dérivé de celui-ci ; l'acide rétinoïque, le α-, γ- ou δ-tocophérol ou le α-, γ- or δ-tocotriénol ; le célécoxib, le rofécoxib, l'étoricoxib, le valdécoxib ou un acide 5-alkyl-2-arylaminophénylacétique, de préférence l'acide 5-méthyl-2-(2'-chloro-6'-fluoroanilino) phénylacétique ; MS-275, SAHA, le pyroxamide, FR-901228 ou l'acide valproïque ; l'acide étridonique, clodronique, tiludronique, pamidronique, alendronique, ibandronique, risédronique et zolédronique ; des inhibiteurs peptidomimétiques et non peptidomimétiques du collagène, des dérivés de la tétracycline, de préférence l'inhibiteur peptidomimétique d'hydroxamate, batimastat, et son analogue biodisponible par voie orale marimastat, le prinomastat, BMS-279251, BAY 12-9566, TAA211 ou AAJ996 ; la rapamycine (sirolimus) ou un dérivé de celle-ci, de préférence la 32-désoxorapamycine, la 16-pent-2-ynyloxy-32-désoxorapamycine, la 16-pent-2-ynyloxy-32 (S)-dihydro-rapamycine, la 16- pent-2-ynyloxy-32(S)-dihydro-40-0-(2-hydroxyéthyl)-rapamycine et, plus préférablement, la 40-0-(2-hydroxyéthyl)-rapamycine ; CCI779 ou 40-(3-hydroxy-2-(hydroxyméthyl)-2-méthylpropanoate]-rapamycine ou un sel pharmaceutiquement acceptable de celui-ci, ABT578 ou la 40-(tétrazolyl)-rapamycine, en particulier la 40-épi-(tétrazolyl)-rapamycine, ou des rapalogues.

14. Combinaison pharmaceutique pour utilisation selon l'une quelconque des revendications 11 à 13, la combinaison étant une combinaison fixe étant administrée sous la forme d'une forme pharmaceutique unique.
